# EUROPÄISCHE PATENTSCHRIFT

(11) **EP 3 389 567 B1**
(45) Veröffentlichungstag und Bekanntmachung des Hinweises auf die Patenterteilung: **30.09.2020**
(21) Anmeldenummer: 16822439.2
(22) Anmeldetag: 16.12.2016
(51) Int. Cl.: A61F 2/28, A61L 27/02, A61L 27/04, A61L 27/06, A61L 27/20, A61L 27/36, A61L 27/58, A61F 2/30, A61F 2/44

(54) **MEDIZINISCHES PRODUKT SOWIE MEDIZINISCHES KIT ZUR ANWENDUNG BEI DER BEHANDLUNG, INSBESONDERE ZUR ANWENDUNG BEIM AUFFÜLLEN UND/ODER VERSCHLUSS, EINER KNOCHENKAVITÄT**
MEDICAL PRODUCT AND MEDICAL KIT FOR USE IN THE TREATMENT, IN PARTICULAR FOR USE IN THE FILLING AND / OR CLOSURE, OF A BONE CAVITY
PRODUITS MEDICAUX ET KIT MÉDICAL POUR UTILISATION DANS LE TRAITEMENT, EN PARTICULIER POUR UNE UTILISATION LORS DE REMPLISSAGE ET / OU DE CLÔTURE, UNE CAVITÉ OSSEUSE

(30) Priorität: 18.12.2015 DE 102015226063
(43) Veröffentlichungstag der Anmeldung: 24.10.2018
(73) Patentinhaber: Aesculap AG, 78532 Tuttlingen (DE)
(72) Erfinder: MATTES, Ursula, 78603 Renquishausen (DE); UTZ, Michael, 78532 Tuttlingen (DE)
(74) Vertreter: Patentanwälte Ruff, Wilhelm, Beier, Dauster & Partner mbB
(86) Internationale Anmeldenummer: PCT/EP2016/081591
(87) Internationale Veröffentlichungsnummer: WO 2017/103216

(56) Entgegenhaltungen:
- EP-A1- 0 366 018
- EP-A1- 0 526 682
- EP-A1- 0 631 763
- WO-A2-2005/094735
- WO-A2-2005/114120
- DE-A1-102006 055 432
- DE-A1-102012 213 246

## Beschreibung

### ANWENDUNGSGEBIET UND STAND DER TECHNIK

Die Erfindung betrifft ein medizinisches Produkt sowie ein Verfahren zur Herstellung des medizinischen Produkts.

Vor allem bei Revisionen nach einer totalen Hüft- oder Kniearthroplastik besteht häufig der Bedarf, kavitäre Knochendefekte aufzufüllen. Die Auffüllung von Knochendefekten ist teilweise auch im Bereich der Wirbelsäulen- und Traumachirurgie erforderlich.

Insbesondere bei osteoporotischen und tumorbefallenen Knochen gestaltet sich die Auffüllung von kavitären Knochendefekten jedoch häufig schwierig.

Zur intraoperativen Auffüllung von Knochenkavitäten stehen heute mehrere Behandlungsoptionen zur Verfügung.

Eine Behandlungsoption besteht in der Verwendung von patienteneigenem Knochen. Alternativ können Knochen aus einer Knochenbank verwendet werden. Stehen beide Optionen nicht zur Verfügung, muss entweder ein metallisches Knochenersatzmaterial oder ein zementartiges Knochenersatzmaterial, wie beispielsweise Kalziumphosphat, Hydroxylapatit oder dergleichen, herangezogen werden. Diese Materialien liegen in der Regel entweder als hartes, gepresstes Formteil oder als loses Pulver vor. Die Adaption dieser festen, künstlichen Knochenersatzmaterialien ist intraoperativ sehr schwierig bis unmöglich, so dass häufig eine vollständige Auffüllung von Knochenkavitäten nicht realisiert werden kann und Hohlräume in den Kavitäten verbleiben.

Ein weiterer Nachteil besteht darin, dass gattungsgemäße Knochenersatzmaterialien häufig nur unzureichend in Knochenkavitäten mit kleinen Zugangsöffnungen implantiert werden können. Pulverförmige Knochenersatzmaterialien lassen sich zwar besser in derartige Kavitäten einbringen. Allerdings besitzen pulverförmige Knochenersatzmaterialien den Nachteil, dass sie nur über begrenzt lasttragende Eigenschaften verfügen.

Aus der DE 10 2006 055 432 A1 ist ein Bauteil mit einem Geflecht aus mehreren, ineinander greifenden, geschlossenen Geflechtelementen bekannt, wobei einzelne Geflechtelemente derart verbunden sind, dass das Geflecht in mindestens einer Raumrichtung schubstabil ist.

### AUFGABE UND LÖSUNG

Der vorliegenden Erfindung lag daher die Aufgabe zugrunde, ein medizinisches Produkt bereitzustellen, welches insbesondere zur Behandlung von Knochenkavitäten geeignet ist und insbesondere Nachteile von aus dem Stand der Technik bekannten Knochenersatzimplantaten sowie -materialien möglichst weitgehend vermeidet.

Weiterhin lag der Erfindung die Aufgabe zugrunde, ein Verfahren zum Herstellen des medizinischen Produkts bereitzustellen.

Diese Aufgaben werden gelöst durch ein medizinisches Produkt gemäß unabhängigem Anspruch 1 sowie durch ein Verfahren gemäß Anspruch 14. Bevorzugte Ausführungsformen sind in den abhängigen Ansprüchen definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung ein medizinisches Produkt, vorzugsweise zur Anwendung bei der Behandlung, insbesondere zur Anwendung beim Auffüllen und/oder Verschluss, einer menschlichen oder tierischen Knochenkavität.

Das medizinische Produkt weist eine Mehrzahl von miteinander verbundenen Gliedern auf. Vorzugsweise sind die Glieder ohne Gelenke oder Befestigungsmittel, wie beispielsweise Hülsen, miteinander verbunden. Jedes Glied weist eine umlaufende Berandung auf, wobei die Berandungen benachbarter Glieder ineinandergreifen oder ineinander eingreifen bzw. ineinandergefügt sind.

Die Glieder weisen vorzugsweise jeweils eine durchgehende Öffnung bzw. ein durchgehendes Loch und/oder einen durchgehenden Kanal auf, welche bzw. welches/welcher von der Berandung umschlossen wird. Anders ausgedrückt, wird durch die Gliederberandung vorzugsweise eine durchgehende Öffnung bzw. ein durchgehendes Loch oder ein durchgehender Kanal definiert.

Unter dem Ausdruck "Knochenkavität" soll im Sinne der vorliegenden Erfindung ein Hohlraum in einem menschlichen oder tierischen Knochen, insbesondere in einem menschlichen oder tierischen Gelenksknochen, vorzugsweise Hüftgelenks- oder Kniegelenksknochen, oder Wirbelkörper, verstanden werden. Der Hohlraum kann die Folge eines Knochentraumas, einer Knochenerkrankung oder einer chirurgischen Intervention/Reintervention, insbesondere einer Revision nach einer totalen Hüft- oder Kniearthroplastik, sein. Bei der Knochenkavität kann es sich insbesondere um eine geschlossene Knochenkavität ("contained defect") oder um eine offene Knochenkavität ("non-contained defect") handeln.

Unter dem Ausdruck "durchgehende Öffnung" bzw. "durchgehendes Loch" soll im Sinne der vorliegenden Erfindung eine Öffnung bzw. ein Loch ohne räumliche Unterbrechung verstanden werden. Entsprechend soll unter dem Ausdruck "durchgehender Kanal" im Sinne der vorliegenden Erfindung ein Kanal ohne räumliche Unterbrechung verstanden werden.

Bevorzugt bilden die Glieder eine stabile, lasttragende Struktur, welche, bedingt durch die Öffnungen bzw. Kanäle der Glieder, eine vorzugsweise osteoinduktive Porosität besitzt.

Ein Vorteil des medizinischen Produkts besteht darin, dass es die Möglichkeit einer Schraubenfixierung bietet. Wird beispielsweise eine Knochenschraube in eine durch die Glieder gebildete Struktur eingedreht, so wirkt die Struktur wie ein Spreizdübel und bietet die Möglichkeit einer mechanischen Befestigung, beispielsweise von Knochenplatten oder Marknägeln.

Das medizinische Produkt bietet weiterhin den Vorteil, dass es in der Lage ist, Knochenkavitäten (im Wesentlichen) vollständig, wenigstens jedoch weitgehend auszufüllen. Dies gilt insbesondere für Knochenkavitäten mit einer internen Hinterschneidung. Eine Anpassung der aufzufüllenden Knochenkavitäten ist somit nicht erforderlich, wodurch das Volumen und insbesondere die Öffnung der Knochenkavitäten möglichst klein gehalten werden kann.

In einer Ausführungsform greifen die Berandungen benachbarter Glieder derart in einander ein, dass sie jeweils in eine durchgehende Öffnung, in ein durchgehendes Loch oder in einen durchgehenden Kanal von wenigstens einem benachbarten Glied eingreifen.

In einer weiteren Ausführungsform weist das medizinische Produkt eine Mehrzahl von Gliedern auf, deren Berandungen jeweils in die Berandung von wenigstens drei weiteren Gliedern, insbesondere von wenigstens vier weiteren Gliedern, eingreifen. Vorzugsweise weist das medizinische Produkt eine Mehrzahl von Gliedern auf, deren Berandungen jeweils in eine durchgehende Öffnung, in ein durchgehendes Loch oder in einen durchgehenden Kanal von wenigstens drei weiteren Gliedern, insbesondere von wenigstens vier weiteren Gliedern, eingreifen.

In einer weiteren Ausführungsform ist eine Mehrzahl von innen liegenden Gliedern mit jeweils einer Mehrzahl von weiteren Gliedern verbunden, wobei das medizinische Produkt außenseitig von einer Anzahl von Bereichen mit jeweils entlang seiner Oberfläche miteinander verbundenen Gliedern begrenzt wird.

Das medizinische Produkt weist eine aus den Gliedern aufgebaute dreidimensionale Struktur auf.

Bevorzugt ist das medizinische Produkt eine aus den Gliedern aufgebaute dreidimensionale Struktur. Mit anderen Worten liegt das medizinische Produkt bevorzugt in Form einer aus den Gliedern aufgebauten dreidimensionalen Struktur vor. Die dreidimensionale Struktur besitzt weiterhin einen mehrlagigen bzw. mehrschichtigen Aufbau, wobei jede Lage bzw. Schicht miteinander verbundene Glieder aufweist, wobei die Berandungen von Gliedern benachbarter Lagen bzw. Schichten ineinandergreifen.

Bei der in den beiden vorherigen Absätzen erwähnten dreidimensionalen Struktur handelt es sich vorzugsweise um eine dreidimensionale Kettenstruktur.

In einer weiteren Ausführungsform ist das medizinische Produkt, insbesondere eine aus den Gliedern aufgebaute Struktur, begrenzt deformierbar.

In einer weiteren Ausführungsform ist das medizinische Produkt, insbesondere eine aus den Gliedern aufgebaute Struktur, reversibel deformierbar.

In einer weiteren Ausführungsform ist das medizinische Produkt, insbesondere eine aus den Gliedern aufgebaute Struktur, fließfähig deformierbar.

In einer weiteren Ausführungsform ist das medizinische Produkt, insbesondere eine aus den Gliedern aufgebaute Struktur, in eine polyederförmige Struktur überführbar. Vorzugsweise ist das Produkt, insbesondere eine aus den Gliedern aufgebaute Struktur, zu einer polyederförmigen Struktur auseinanderziehbar oder expandierbar bzw. entfaltbar. Bei der polyederförmigen Struktur kann es sich beispielsweise um eine würfelförmige, quaderförmige, prismenförmige, pyramidenförmige, spatenförmige oder um eine aus Freiformflächen begrenzte Struktur handeln.

In einer alternativen Ausführungsform ist das medizinische Produkt, insbesondere eine aus den Gliedern aufgebaute Struktur, in eine nicht-polyederförmige Struktur überführbar. Vorzugsweise ist das Produkt, insbesondere eine aus den Gliedern aufgebaute Struktur, zu einer nichtpolyederförmigen Struktur auseinanderziehbar oder expandierbar bzw. entfaltbar. Bei der nichtpolyederförmigen Struktur kann es sich beispielsweise um eine kugelförmige, ellipsoide, toroidförmige oder kegelförmige Struktur handeln.

Wenigstens ein Teil der Glieder weist in einer weiteren Ausführungsform jeweils eine geschlossene Berandung, d.h. eine unterbrechungsfreie bzw. ununterbrochene Berandung (Berandung ohne Unterbrechungen), auf. Erfindungsgemäß kann es insbesondere vorgesehen sein, dass die Berandungen von allen Gliedern des medizinischen Produkts jeweils geschlossen sind.

Der Ausdruck "wenigstens ein Teil der Glieder" kann im Sinne der vorliegenden Erfindung ein Glied, mehrere Glieder oder alle Glieder des medizinischen Produkts bedeuten.

In einer weiteren Ausführungsform weist wenigstens ein Teil der Glieder jeweils wenigstens eine, insbesondere nur eine, Öffnung bzw. Unterbrechung, insbesondere wenigsten einen, insbesondere nur einen, Spalt auf. Erfindungsgemäß kann es insbesondere vorgesehen sein, dass die Berandungen von allen Gliedern des medizinischen Produkts jeweils wenigstens eine, insbesondere nur eine, Öffnung bzw. Unterbrechung, insbesondere wenigstens einen, insbesondere nur einen, Spalt aufweisen. Auf diese Weise können einzelne Glieder voneinander getrennt und das medizinische Produkt in Form und Größe an eine zu behandelnde Knochenkavität angepasst werden.

In einer weiteren Ausführungsform weist wenigstens ein Teil der Glieder jeweils wenigstens eine, insbesondere nur eine, Sollbruchstelle auf. Die Sollbruchstelle kann beispielsweise als Kerbe, poröse Struktur, Perforation oder Materialeinschluss ausgestaltet sein. Erfindungsgemäß kann es insbesondere vorgesehen sein, dass die Berandungen von allen Gliedern des medizinischen Produkts jeweils wenigstens eine, insbesondere nur eine, Sollbruchstelle aufweisen. Auf diese Weise können einzelne Glieder voneinander getrennt und das medizinische Produkt, insbesondere eine aus den Gliedern aufgebaute Struktur, verkleinert und besser an eine zu behandelnde Knochenkavität angepasst werden.

Die Glieder oder Berandungen können grundsätzlich jede beliebige Form, Geometrie oder Außenkontur, insbesondere eine aus Freiformflächen erstellte, insbesondere gedruckte, Geometrie oder Form, aufweisen.

Insbesondere können die Glieder oder Berandungen polygyonal und/oder nicht-polygonal und/oder polyederförmig und/oder nicht-polyederförmig ausgebildet sein.

Beispielsweise können die Glieder oder Berandungen dreieckig, viereckig, fünfeckig, sechseckig, siebeneckig, achteckig, neuneckig, zehneckig, kreisförmig, ovalförmig, ellipsenförmig, ringförmig, toroidförmig, quaderförmig, würfelförmig, prismenförmig, pyramidenförmig, spatenförmig oder sternförmig ausgebildet sein.

Weiterhin können die Glieder oder Berandungen unterschiedlich ausgestaltet sein. Bezüglich möglicher Ausgestaltungen der Glieder bzw. Berandungen wird insbesondere auf die im letzten Absatz beschriebenen Ausgestaltungen Bezug genommen.

Die Glieder weisen in einer weiteren Ausführungsform jeweils einen lichten Durchmesser (Innendurchmesser) von 0.1 mm bis 20 mm, insbesondere 1.5 mm bis 5 mm, bevorzugt 2 mm bis 4 mm, auf.

Die Berandungen weisen gemäß einer weiteren Ausführungsform jeweils eine Stärke bzw. Dicke (Berandungsstärke bzw. -dicke) von 0.2 mm bis 5 mm, insbesondere 0.5 mm bis 4 mm, bevorzugt 1 mm bis 3 mm, auf.

Gemäß einer weiteren Ausführungsform sind die Glieder des medizinischen Produkts in zwei oder mehr Gruppen einteilbar, wobei die Glieder jeder Gruppe identisch zueinander ausgebildet sind und die Glieder unterschiedlicher Gruppen unterschiedlich zueinander ausgebildet sind.

Vorzugsweise unterscheiden sich die Glieder unterschiedlicher Gruppen hinsichtlich wenigstens einer Eigenschaft, welche ausgewählt ist aus der Gruppe aufweisend oder bestehend aus Gesamtdurchmesser, lichter Durchmesser, Größe, Form, Geometrie, Außenkontur, Material, Berandungsstärke bzw. -dicke, Farbe, Additivierung wie Wirkstoffadditivierung und Kombinationen aus zwei oder mehreren der genannten Eigenschaften.

Erfindungsgemäß kann es weiterhin bevorzugt sein, dass manche Glieder, insbesondere Glieder an Rand- und/oder Kanten- und/oder Eckbereichen des medizinischen Produkts, einen größeren lichten Durchmesser aufweisen als andere Glieder des Produkts, insbesondere innere Glieder des Produkts. Auf diese Weise ist mit besonderem Vorteil eine Befestigung des Produkts auch an/in offenen Knochenkavitäten, das heißt sogenannten "non-contained defects" oder an Implantaten möglich.

In einer weiteren Ausführungsform weisen manche Glieder, insbesondere Glieder an Rand- und/oder Kanten- und/oder Eckbereichen des Produkts, eine Markierung, insbesondere farbige und/oder geometrische Markierung, auf. Dies kann es beispielsweise einem Anwender, in der Regel einem Chirurgen, erleichtern, das medizinische Produkt auseinanderzuziehen bzw. zu entfalten.

Weiterhin kann es erfindungsgemäß bevorzugt sein, dass manche Glieder, insbesondere Glieder an Rand- und/oder Kanten- und/oder Eckbereichen des medizinischen Produkts, ein Greifelement aufweisen. Das Greifelement kann beispielsweise kugelförmig oder würfelförmig ausgestaltet sein. Das Greifelement erleichtert mit besonderem Vorteil die Handhabung für den Anwender. Das Greifelement kann weiterhin monolithisch bzw. einstückig mit den entsprechenden Gliedern verbunden sein. Beispielsweise kann das Greifelement an die entsprechenden Glieder angeformt sein. Alternativ kann das Greifelement stoffschlüssig mit den entsprechenden Gliedern verbunden sein. Zum Beispiel kann das Greifelement mit den entsprechenden Gliedern verklebt oder verschweißt sein.

Ein Teil der Glieder ist derart miteinander verbunden, dass sie nicht relativ zueinander beweglich sind. Dadurch kann das medizinische Produkt, insbesondere eine aus den Gliedern aufgebaute Struktur, bevorzugt Ecken und/oder Kanten und/oder Flächen einer solchen Struktur, stabiler ausgestaltet werden. Bevorzugt sind Glieder an Rand- und/oder Kanten- und/oder Eckbereichen des Produkts, insbesondere einer aus den Gliedern aufgebauten Struktur, derart miteinander verbunden, dass sie nicht relativ zueinander beweglich sind. Die Glieder sind vorzugsweise stoffschlüssig miteinander verbunden, insbesondere verklebt und/oder verschweißt. Alternativ oder in Kombination können die Glieder einteilig bzw. einstückig (monolithisch) ausgestaltet sein. Die Glieder sind mittels eines additiven bzw. generativen Fertigungsverfahrens hergestellt, insbesondere gedruckt. Bezüglich geeigneter additiver bzw. generativer Fertigungsverfahren wird auf die im Folgenden noch beschriebenen Fertigungsverfahren Bezug genommen.

Grundsätzlich bietet die Auswahl des Materials für die Glieder oder Berandungen eine weitere Möglichkeit, um die Eigenschaften des medizinischen Produkts zu beeinflussen.

Bezüglich in Frage kommender Materialien für die Glieder oder Berandungen bestehen grundsätzlich keine Limitierungen, sofern es sich um bioverträgliche und somit medizinisch verwendbare bzw. patientenverträgliche Materialien handelt.

In einer weiteren Ausführungsform weisen die Glieder oder Berandungen ein nicht resorbierbares, teilresorbierbares oder resorbierbares Material auf oder bestehen aus einem solchen Material. Bezüglich geeigneter Materialien sei auf die nachfolgenden Ausführungen verwiesen.

In einer weiteren Ausführungsform weisen die Glieder ein Material auf oder bestehen aus einem Material, welches ausgewählt ist aus der Gruppe bestehend aus Metalle, Polymere, Keramikmaterialien, osteokonduktive Materialien, Knochenzementmaterialien und Mischungen bzw. Kombinationen aus zwei oder mehreren der genannten Materialien.

Die im vorherigen Absatz erwähnten Metalle sind bevorzugt ausgewählt aus der Gruppe bestehend aus Edelstahl, Magnesium, Tantal, Titan, Chrom, Kobalt und Kombinationen, insbesondere Legierungen, aus zwei oder mehreren der genannten Metalle. Geeignete Legierungen stellen beispielsweise Kobalt-Chrom-Legierungen dar. Die Verwendung von Metallen erlaubt mit besonderem Vorteil die Herstellung von dauerhaften medizinischen Produkten.

Bei den oben erwähnten Polymeren kann es sich grundsätzlich um resorbierbare, teilresorbierbare oder nicht-resorbierbare Polymere handeln. Durch die Verwendung von resorbierbaren oder teilresorbierbaren Polymeren lassen sich mit besonderem Vorteil osteoinduktive Produkte herstellen, welche das Knochenwachstum in die Knochenkavitäten fördern.

Bevorzugt sind die Polymere ausgewählt aus der Gruppe bestehend aus Polyolefine, Polyester, Polyamide, Polyhydroxyalkanoate, Proteine wie extrazelluläre Proteine, Polysaccharide wie Cellulosederivate und/oder Mucopolysaccharide, Stereoisomere, insbesondere Diastereomere, davon, Salze davon, Copolymere davon und Mischungen aus zwei oder mehreren der genannten Polymere.

Gemäß einer weitergehenden Ausführungsform können die Polymere ausgewählt sein aus der Gruppe bestehend aus Polyolefine, Polyethylen (wie Polyethylen niedriger Dichte, Polyethylen hoher Dichte und/oder ultrahochmolekulares Polyethylen (UHMWPE)), Polypropylen, Polytetrafluorethylen, Polyvinylidenchlorid, Polyvinylidenfluorid, Polytetrafluorpropylen, Polyhexafluorpropylen, Polyacrylat, Polymethylacrylat, Polymethylmethacrylat, Polyester, Polyethylenterephthalat, Polypropylenterephthalat, Polybutylenterephthalat, Polyamide, Polyamid 6, Polyamid 6-6, Polyamid 6-12, Polyamid 12, Seide, Polymilchsäure bzw. Polylactid, Polyglykolsäure bzw. Polyglykolid, Poly-3-hydroxybutyrat, Poly-4-hydroxybutyrat, Polytrimethylencarbonat, Poly-ε-Caprolacton, extrazelluläre Proteine, Collagen, Gelatine, Elastin, Retikulin, Fibronectin, Laminin, Fibrin, Fibrinogen, Albumin wie Serumalbumin, Stärke, Amylose, Amylopectin, Dextran, Dextrin, Cellulose, Cellulosederivate wie Alkylcellulose, Hydroxyalkylcellulose, Hydroxymethylcellulose, Hydroxyethylcellulose, Hydroxypropylcellulose, Carboxyalkylcellulose, Carboxymethylcellulose, Chitin, Chitosan, Hyaluronsäure, Dextransulfat, Heparin, Heparansulfat, Chondroitinsulfat, Dermatansulfat, Salze davon, Stereoisomere, insbesondere Diastereomere, davon, Copolymere davon und Mischungen aus zwei oder mehreren der genannten Polymere.

In einer weiteren Ausführungsform sind die oben genannten osteokonduktiven Materialien ausgewählt aus der Gruppe bestehend aus Hydroxylapatit, α-Tricalciumphosphat, β-Tricalciumphosphat und Mischungen aus wenigstens zwei der genannten osteokonduktiven Materialien.

In einer weiteren Ausführungsform weisen die oben genannten Knochenzementmaterialien eine Calcium- und/oder Magnesiumverbindung auf, welche ausgewählt ist aus der Gruppe bestehend aus Monocalciumphosphat-Monohydrat (MCPM), Monocalciumphosphat-Anhydrid (MCPA), Dicalciumphosphat-Anhydrid (DCPA), Dicalciumphosphat-Dihydrat (DCPD), Octacalciumphosphat (OCP), α-Tricalciumphosphat (a-TCP), β-Tricalciumphosphat (β-TCP), amorphes Calciumphosphat (ACP), Hydroxylapatit (HA), Calcium-defizientes Hydroxylapatit (CdHA), substituiertes Hydroxylapatit, nicht stöchiometrisches Hydroxylapatit, nanoskaliges Hydroxylapatit, Tetracalciumphosphat (TTCP), Calciumsulfat (CaSO₄), Calciumsulfat-Hemihydrat (CaSO₄ x 0.5 H₂O), Calciumsulfat-Dihydrat (CaSO₄ x 2 H₂O), Calciumoxid (CaO), Calciumhydroxid (Ca(OH)₂), Calciumcarbonat (CaCO₃), Calciumglycerophosphat, Calciumcitrat, Calciumlactat, Calciumacetat, Calciumtartrat, Calciumchlorid (CaCl₂), Calciumsilicate, Magnesiumhydrogenphosphat (MgHPO₄) in Form der Hydrate oder als wasserfreie Substanz, Trimagnesiumphosphat (Mg₃(PO₄)₂), Magnesiumdihydrogenphosphat (Mg(H₂PO₄)₂) in Form der Hydrate oder als wasserfreie Substanz, Magnesiumchlorid (MgCl₂) in Form der Hydrate oder als wasserfreie Substanz, Magnesiumglycerophosphat, Magnesiumhydroxid (Mg(OH)₂), Magnesiumhydroxidcarbonat (beispielsweise als 4 MgCO₃ x Mg(OH)₂ x 5 H₂O), Magnesiumoxid (MgO), Magnesiumcitrate (Mg₃(C₆H₅O₇)₂) oder Mg(C₆H₆O₇)), Calcium-Magnesiumcarbonat (CaMg(CO₃)₂, Dolomit und Mischungen aus zwei oder mehreren der genannten Verbindungen.

In einer weiteren Ausführungsform weist das medizinische Produkt neben den Gliedern zusätzliche Strukturelemente auf.

Die zusätzlichen Strukturelemente sind vorzugsweise zwischen den Gliedern des medizinischen Produkts angeordnet.

Bevorzugt sind die zusätzlichen Strukturelemente im Inneren des medizinischen Produkts angeordnet oder integriert.

Besonders bevorzugt befinden sich die zusätzlichen Strukturelemente in Hohl- und/oder Zwischenräumen des medizinischen Produkts, insbesondere in Hohl- und/oder Zwischenräumen einer aus den Gliedern aufgebauten Struktur.

Insbesondere können Hohl- und/oder Zwischenräume des medizinischen Produkts, insbesondere Hohl- und/oder Zwischenräume einer aus den Gliedern aufgebauten Struktur, nur teilweise mit den zusätzlichen Strukturelementen belegt sein.

Die zusätzlichen Strukturelemente stellen mit besonderem Vorteil neben den Gliedern eine weitere Möglichkeit dar, die Eigenschaften des medizinischen Produkts gezielt einzustellen. Beispielsweise kann mittels der zusätzlichen Strukturelemente die mechanische Stabilität, die Steifigkeit oder Elastizität, die in-vivo-Stabilität oder Resorption, das Fließverhalten oder die Osteokonduktivität des medizinischen Produkts gezielt gesteuert oder kontrolliert werden.

In einer weiteren Ausführungsform sind die zusätzlichen Strukturelemente größer ausgebildet als die Glieder. Bevorzugt weisen die zusätzlichen Strukturelemente wenigstens eine größere Abmessung auf als die Glieder. Bei der wenigstens einen größeren Abmessung kann es sich insbesondere um die Länge und/oder die Dicke bzw. Höhe und/oder den Durchmesser, insbesondere Innen- und/oder Außendurchmesser, der zusätzlichen Strukturelemente handeln.

In einer weiteren Ausführungsform sind die zusätzlichen Strukturelemente größer ausgebildet als Hohl- und/oder Zwischenräume des medizinischen Produkts, insbesondere Hohl- und/oder Zwischenräume einer aus den Gliedern aufgebauten Struktur. Bevorzugt weisen die zusätzlichen Strukturelemente wenigstens eine größere Abmessung auf als Hohl- und/oder Zwischenräume des medizinischen Produkts, insbesondere Hohl- und/oder Zwischenräume einer aus den Gliedern aufgebauten Struktur. Bei der wenigstens einen größeren Abmessung kann es sich insbesondere um die Länge und/oder die Dicke bzw. Höhe und/oder den Durchmesser, insbesondere Innen- und/oder Außendurchmesser, der zusätzlichen Strukturelemente handeln. Durch die in diesem Absatz beschriebenen Ausführungsformen kann mit besonderem Vorteil eine Aussteifung des medizinischen Produkts realisiert werden.

Grundsätzlich können die zusätzlichen Strukturelemente jede beliebige Form, Geometrie oder Außenkontur, insbesondere eine aus Freiformflächen erstellte, insbesondere gedruckte, Geometrie oder Form, aufweisen.

In einer weiteren Ausführungsform liegen die zusätzlichen Strukturelemente als Formkörper vor. Mit anderen Worten sind die zusätzlichen Strukturelemente gemäß einer weiteren Ausführungsform regelmäßig geformt. Unter dem Ausdruck "regelmäßig geformt" sollen im Sinne der vorliegenden Erfindung insbesondere die nachfolgend beschriebenen Formen verstanden werden.

Die zusätzlichen Strukturelemente, insbesondere Formkörper, können einen polygonen, insbesondere dreieckförmigen, quadratförmigen, rechteckförmigen, fünfeckförmigen, sechseckförmigen, siebeneckförmigen, achteckförmigen, neuneckförmigen, zehneckförmigen oder sternenförmigen, Querschnitt aufweisen.

Insbesondere können die zusätzlichen Strukturelemente, insbesondere Formkörper, unterschiedliche Querschnitte aufweisen. Bezüglich möglicher Querschnitte wird auf die im vorherigen Absatz genannten Querschnitte Bezug genommen.

Weiterhin können die zusätzlichen Strukturelemente, insbesondere Formkörper, polyederförmig, insbesondere quader-, würfel-, tetraeder-, prismen-, pyramiden-, pyramidenstumpf- oder spatförmig, ausgebildet sein.

Weiterhin können die zusätzlichen Strukturelemente, insbesondere Formkörper, unterschiedlich polyederförmig ausgebildet sein. Mit anderen Worten können die zusätzlichen Strukturelemente, insbesondere Formkörper, in unterschiedlichen Polyederformen vorliegen. Bezüglich möglicher polyederförmiger Ausbildungen wird auf den vorherigen Absatz Bezug genommen.

Weiterhin können die zusätzlichen Strukturelemente, insbesondere Formkörper, einen eckenlosen Querschnitt aufweisen. Beispielsweise können die zusätzlichen Strukturelemente, insbesondere Formkörper, einen ovalförmigen, insbesondere kreisförmigen oder elliptischen, Querschnitt aufweisen.

Weiterhin können die zusätzlichen Strukturelemente, insbesondere Formkörper, nicht-polyederförmig, insbesondere kugel-, kegel-, kegelstumpf-, ring-, toroid- oder kreiszylinderförmig, ausgebildet sein.

Beispielsweise können die zusätzlichen Strukturelemente kugelförmig ausgebildet sein. Abhängig von der Größe der zusätzlichen Strukturelemente lassen sich hierbei unterschiedliche Effekte erzielen. Sind die kugelförmigen Strukturelemente beispielsweise (deutlich) kleiner ausgebildet als Hohl- und/oder Zwischenräume des medizinischen Produkts, insbesondere Hohl- und/oder Zwischenräume einer aus den Gliedern aufgebauten Struktur, ist keine Stabilisierung oder allenfalls eine Stabilisierung des medizinischen Produkts in eine Raumrichtung erzielbar. Abhängig von den Eigenschaften kugelförmig ausgebildeter Strukturelemente können dem medizinischen Produkt jedoch zusätzliche Eigenschaften verliehen werden, ohne beispielsweise ein "Fließvermögen" des medizinischen Produkts (nennenswert) zu beeinflussen. Werden Hohl- und/oder Zwischenräume des medizinischen Produkts, insbesondere Hohl- und/oder Zwischenräume einer aus den Gliedern aufgebauten Struktur, dagegen nahezu komplett ausgefüllt oder sind die kugelförmigen Strukturelemente größer ausgebildet als Hohl- und/oder Zwischenräume des medizinischen Produkts, insbesondere Hohl- und/oder Zwischenräume einer aus den Gliedern aufgebauten Struktur, so kann hierdurch beispielsweise eine stabilisierende/aussteifende Wirkung erzielt werden. Weisen die zusätzlichen Strukturelemente ein resorbierbares Material auf oder bestehen die zusätzlichen Strukturelemente aus einem solchen Material, kann mit besonderem Vorteil eine zeitabhängige Stabilisierung/Aussteifung des medizinischen Produkts erreicht werden, wobei die Stabilisierung/Aussteifung mit der Zeit abnimmt.

Weiterhin können die zusätzlichen Strukturelemente, insbesondere Formkörper, unterschiedlich nicht-polyederförmig ausgebildet sein. Mit anderen Worten können die zusätzlichen Strukturelemente, insbesondere Formkörper, in unterschiedlichen Nicht-Polyederformen vorliegen. Bezüglich möglicher nicht-polyederförmiger Ausbildungen wird auf den vorherigen Absatz Bezug genommen.

Weiterhin können die zusätzlichen Strukturelemente, insbesondere Formkörper, als Oligopoden ausgebildet sein. Die Oligopoden können ausgewählt sein aus der Gruppe bestehend aus Tripoden, Tetrapoden, Pentapoden, Hexapoden, Heptapoden, Oktapoden und Mischungen aus wenigstens zwei der genannten Oligopoden.

Weiterhin können die zusätzlichen Strukturelemente, insbesondere Formkörper, ausgewählt sein aus der Gruppe bestehend aus Polyeder, Nicht-Polyeder, Oligopoden und Kombinationen davon. Bezüglich möglicher Polyeder-, Nicht-Polyeder- sowie Oligopodenausgestaltungen wird auf die vorherigen Absätze Bezug genommen.

In einer weiteren Ausführungsform sind die zusätzlichen Strukturelemente sternenförmig ausgebildet. Auf diese Weise können mit besonderem Vorteil die miteinander verbundenen Glieder stabilisiert und ein Abrutschen der Glieder gegeneinander reduziert werden. Dadurch kann die "Fließfähigkeit" des medizinischen Produkts vermindert und dessen Steifigkeit erhöht werden.

In einer weiteren Ausführungsform sind die zusätzlichen Strukturelemente unregelmäßig geformt.

In einer bevorzugten Ausführungsform sind die zusätzlichen Strukturelemente nicht mit den Gliedern verbunden. Bei dieser Ausführungsform kann es zweckmäßig sein, wenn die zusätzlichen Strukturelemente in etwa so groß ausgebildet sind wie Hohl- und/oder Zwischenräume des medizinischen Produkts, insbesondere Hohl- und/oder Zwischenräume einer aus den Gliedern aufgebauten Struktur, oder sogar größer ausgebildet sind als Hohl- und/oder Zwischenräume des medizinischen Produkts, insbesondere Hohl- und/oder Zwischenräume einer aus den Gliedern aufgebauten Struktur. Dadurch kann mit besonderem Vorteil eine unbeabsichtigte Freisetzung der zusätzlichen Strukturelemente aus dem medizinischen Produkt verhindert werden.

In einer weiteren Ausführungsform sind die zusätzlichen Strukturelemente mit den Gliedern des medizinischen Produkts, insbesondere mit wenigstens einem Teil der Glieder, verbunden. Die Verbindung zwischen den zusätzlichen Strukturelementen und den Gliedern kann beispielsweise auf einer stoffschlüssigen Verbindung, insbesondere auf einer Klebe- und/oder Schweißverbindung, beruhen. Alternativ können die zusätzlichen Strukturelemente und die Glieder einstückig bzw. einteilig (monolithisch) ausgebildet sein. Insbesondere können die zusätzlichen Strukturelemente an die Glieder des medizinischen Produkts angeformt sein. Eine einstückige Ausbildung oder eine Anformung ist beispielsweise mittels eines additiven bzw. generativen Fertigungsverfahrens möglich. Bezüglich geeigneter additiver bzw. generativer Fertigungsverfahren wird vollständig auf die noch folgende Beschreibung bezuggenommen.

In einer weiteren Ausführungsform sind die zusätzlichen Strukturelemente länglich, insbesondere stäbchen-, draht-, faden- oder schnurförmig, ausgebildet. Insbesondere kann es sich bei den zusätzlichen Strukturelementen beispielsweise um Fäden, insbesondere chirurgische Fäden, handeln.

In einer weiteren Ausführungsform sind die zusätzlichen Strukturelemente als Flächengebilde ausgebildet. Bei den Flächengebilden kann es sich um textile Flächengebilde, insbesondere Netze, oder nicht-textile Flächengebilde, insbesondere Gitter und/oder Platten, handeln. Die Ausbildung der zusätzlichen Strukturelemente als Flächengebilde hat den Vorteil, dass eine Stabilisierung, insbesondere Versteifung, des medizinischen Produkts allenfalls in eine Raumrichtung erzeugt wird, so dass das medizinische Produkt über derart ausgestaltete Strukturelemente gezielt mit Eigenschaften ausgestattet werden kann, ohne eine "Fließfähigkeit" des medizinischen Produkts (nennenswert) zu beeinflussen.

Grundsätzlich können die zusätzlichen Strukturelemente jedes beliebige Material aufweisen oder aus jedem beliebigen Material bestehen, sofern es sich bei dem Material um ein bioverträgliches und mithin patientenverträgliches Material handelt. Bezüglich geeigneter Materialien wird vollständig auf die im Zusammenhang der Glieder beschriebenen Materialien Bezug genommen. Die dort beschriebenen Metalle, Polymere, Keramikmaterialien, osteokonduktiven Materialien sowie Knochenzementmaterialien können somit auch für die zusätzlichen Strukturelemente verwendet werden.

Weiterhin können die zusätzlichen Strukturelemente beispielsweise ein Metall aufweisen oder aus einem Metall bestehen, welches ausgewählt ist aus der Gruppe bestehend auf Edelstahl, Magnesium, Tantal, Titan, Chrom, Kobalt und Kombinationen, insbesondere Legierungen, aus wenigstens zwei der genannten Metalle.

In einer weiteren Ausführungsform weisen die zusätzlichen Strukturelemente ein resorbierbares Material auf oder bestehen aus einem resorbierbaren Material. Die Verwendung eines resorbierbaren Materials hat insbesondere den Vorteil, dass die Stabilisierung des medizinischen Produkts zeitabhängig gestaltet werden kann. Das resorbierbare Material kann beispielsweise ausgewählt sein aus der Gruppe bestehend aus Polyhydroxyalkanoat, Polymilchsäure bzw. Polylactid, Polyglykolsäure bzw. Polyglycolid, Poly-3-Hydroxybutyrat, Poly-4-Hydroxybutyrat, Polytrimethylencarbonat, Poly-ε-Caprolacton, Protein wie extrazelluläres Protein, Gelatine, Collagen, Elastin, Retikulin, Fibronektin, Laminin, Albumin wie Serumalbumin, Stärke, Amylose, Amylopektin, Dextran, Cellulose, Hydroxymethylcellulose, Carboxymethylcellulose, Chitosan, Hyaluronsäure, Heparin, Heparansulfat, Salze davon, Stereoisomere, insbesondere Diastereomere, davon, Copolymere davon und Mischungen aus wenigstens zwei der genannten Materialien.

In einer weiteren Ausführungsform weisen die zusätzlichen Strukturelemente ein nicht resorbierbares Material auf oder bestehen aus einem nicht resorbierbaren Material. Alternativ können die zusätzlichen Strukturelemente ein langzeitstabiles Material aufweisen oder aus einem solchen Material bestehen. Die Verwendung eines nicht resorbierbaren bzw. langzeitstabilen Materials hat den Vorteil, dass das medizinische Produkt wenigstens teilweise eine (im Wesentlichen) zeitlich unbegrenzte Stabilität aufweist. Das nicht resorbierbare bzw. langzeitstabile Material kann beispielsweise ausgewählt sein aus der Gruppe bestehend aus Polyolefin, Polyethylen, ultrahochmolekulares Polyethylen, Polypropylen, Polytetrafluorethylen, Polyvinylidenchlorid, Polyvinylidenfluorid, Polyacrylat, Polymethacrylat, Polymethylmethacrylat, Polyester, Polyethylenterephthalat, Polyamid, Polyamid 6, Polyamid 6-6, Polyamid 6-12, Seide, Stereoisomere, insbesondere Diastereomere, davon, Copolymere davon und Mischungen aus wenigstens zwei der genannten Materialien.

In einer weiteren Ausführungsform weisen die zusätzlichen Strukturelemente ein osteokonduktives Material auf oder bestehen aus einem osteokonduktiven Material. Unter dem Ausdruck "osteokonduktives Material" soll im Sinne der vorliegenden Erfindung ein Material verstanden werden, welches dazu eingerichtet ist, dass Einwachsen von Knochengewebe, insbesondere Knochenneugewebe, in das medizinische Produkt und damit eine knöcherne Integration des medizinischen Produkts zu begünstigen. Das osteokonduktive Material ist vorzugsweise ausgewählt aus der Gruppe bestehend aus Hydroxylapatit, α-Tricalciumphosphat, β-Tricalciumphosphat und Mischungen aus wenigstens zwei der genannten osteokonduktiven Materialien.

In einer weiteren Ausführungsform handelt es sich bei den zusätzlichen Strukturelementen um Knochenpartikel, insbesondere autologe Knochenpartikel. Die Knochenpartikel können insbesondere als Knochenspäne vorliegen.

In einer weiteren Ausführungsform sind die Glieder des medizinischen Produkts wenigstens teilweise, insbesondere vollständig, aus einem resorbierbaren Material, wie beispielsweise einem Polyhydroxyalkanoat, einem Protein, einem Polysaccharid oder aus einer Mischung aus wenigstens zwei der genannten Materialien gebildet. Bezüglich möglicher Polyhydroxyalkanoate, Proteine sowie Polysaccharide wird vollständig auf die bisherige Beschreibung Bezug genommen.

In einer weiteren Ausführungsform weisen die zusätzlichen Strukturelemente ein elastomeres Material auf oder bestehen aus einem elastomeren Material. Dadurch lassen sich beispielsweise Dämpfer- oder Federeigenschaften des medizinischen Produkts verwirklichen. Erfindungsgemäß ist es insbesondere denkbar, dass ein mit elastomeren Strukturelementen versehenes medizinisches Produkt derart reversibel ausgestaltet ist, dass es sich beim Einbringen in eine Knochenkavität komprimieren und nach Einbringen in die Knochenkavität durch Wegnahme einer für die Kompression verantwortlichen Kraft wieder entfalten lässt, wodurch die Knochenkavität mit dem medizinischen Produkt aufgefüllt werden kann. Bei dem elastomeren Material kann es sich insbesondere um ein Silikon und/oder einen Silikonkautschuk, beispielsweise um einen unter der Bezeichnung SILPURAN® kommerziell erhältlichen Silikonkautschuk, handeln.

In einer weiteren Ausführungsform weisen die zusätzlichen Strukturelemente, insbesondere wenigstens ein Teil der zusätzlichen Strukturelemente, einen Wirkstoff auf. Bevorzugt sind die zusätzlichen Strukturelemente, insbesondere wenigstens ein Teil der zusätzlichen Strukturelemente, mit einem Wirkstoff beschichtet. Bezüglich Beispiele für einen geeigneten Wirkstoff sei auf die nachfolgenden Ausführungen Bezug genommen.

In einer weiteren Ausführungsform weist wenigstens ein Teil der Glieder oder Berandungen einen Wirkstoff auf. Bevorzugt ist wenigstens ein Teil der Glieder oder Berandungen mit einem Wirkstoff beschichtet.

Erfindungsgemäß kann es insbesondere vorgesehen sein, dass alle Glieder des medizinischen Produkts oder alle Berandungen des medizinischen Produkts jeweils einen Wirkstoff aufweisen. Bevorzugt sind alle Glieder des medizinischen Produkts oder alle Berandungen des medizinischen Produkts jeweils mit einem Wirkstoff beschichtet.

Der Wirkstoff ist vorzugsweise ausgewählt aus der Gruppe bestehend aus antimikrobieller, insbesondere antibiotischer, Wirkstoff, wundheilungsfördernder Wirkstoff, desinfizierender Wirkstoff, entzündungshemmender bzw. antiinflammatorischer Wirkstoff, blutgerinnungsfördernder Wirkstoff, Wachstumsfaktor wie Knochenwachstumsfaktor (osteoinduktiver Faktor), zelldifferenzierender Faktor, zelladhäsiver Faktor, zellrekrutierender Faktor, Zellrezeptor, zellbindender Faktor, Zytokin, Peptid, Strukturprotein, extrazelluläres Protein wie Collagen, Elastin, Retikulin und dergleichen, Serumprotein wie Albumin, Polysaccharid wie Hyaluronsäure, Oligonukleotid, Polynukleotid, DNA, RNA, Salze davon, Stereoisomere, insbesondere Diastereomere, davon und Mischungen aus zwei oder mehreren der genannten Wirkstoffe.

In einer besonders bevorzugten Ausführungsform handelt es sich bei dem Wirkstoff um einen Knochenwachstumsfaktor, insbesondere um ein knochenmorphogenetisches Protein (BMP). Das knochenmorphogenetische Protein ist vorzugsweise ausgewählt aus der Gruppe bestehend aus knochenmorphogenetisches Protein 1 (BMP1), knochenmorphogenetisches Protein 2 (BMP2), knochenmorphogenetisches Protein 3 (BMP3), knochenmorphogenetisches Protein 3B (BMP3B), knochenmorphogenetisches Protein 4 (BMP4), knochenmorphogenetisches Protein 5 (BMP5), knochenmorphogenetisches Protein 6 (BMP6), knochenmorphogenetisches Protein 7 (BMP7), knochenmorphogenetisches Protein 8A (BMP8A), knochenmorphogenetisches Protein 8B (BMP8B), knochenmorphogenetisches Protein 10 (BMP10), knochenmorphogenetisches Protein 15 (BMP15) und Mischungen aus zwei oder mehreren der genannten knochenmorphogenetischen Proteine.

In einer weiteren Ausführungsform ist das Produkt, insbesondere die Glieder oder die Berandungen, mittels eines additiven bzw. generativen Fertigungsverfahrens hergestellt. Unter dem Ausdruck "additive Fertigungsverfahren" bzw. "generative Fertigungsverfahren" sollen im Sinne der vorliegenden Erfindung Verfahren zur schnellen und kostengünstigen Fertigung von Modellen, Mustern, Prototypen, Werkzeugen und Endprodukten verstanden werden ("additive Fertigung"). Diese Verfahren werden häufig auch als Rapid Prototyping bezeichnet. Die Fertigung erfolgt direkt auf der Basis von rechnerinternen Datenmodellen aus formlosem (Flüssigkeiten, Pulver und Ähnliches) oder formneutralem (band-, drahtförmig) Material mittels chemischer und/oder physikalischer Prozesse. Ein additives bzw. generatives Fertigungsverfahren bietet den Vorteil, Glieder oder Berandungen in unterschiedlichen Dimensionen, Formen, Geometrien und/oder Außenkonturen, insbesondere mit unterschiedlichen Innen- und Außendurchmessern, herzustellen. Insbesondere können mithilfe von additiven Fertigungsverfahren Größe und/oder geometrische Ausprägungen einzelner Glieder innerhalb einer Gliederkette variiert und/oder kombiniert werden. Weiterhin können unterschiedliche Größen und Ausformungen, beispielsweise Ringe, Dreiecke, Sterne, Würfel oder dergleichen, gemischt werden. Additive bzw. generative Fertigungsverfahren erlauben somit in besonderer Weise eine variable Herstellung von Gliedern, wodurch sich die Eigenschaften des medizinischen Produkts gezielt beeinflussen lassen. Beispielweise lassen sich hierdurch Eigenschaften des medizinischen Produkts, wie beispielsweise dessen Steifigkeit, "Fließvermögen", Beweglichkeit sowie Porosität, gezielt an die jeweils zu behandelnde Knochenkavität anpassen. Auch der Grad des "Ineinanderverkeilens" einzelner Glieder lässt sich mithilfe von additiven bzw. generativen Fertigungsverfahren besonders bequem einstellen.

Das additive bzw. generative Fertigungsverfahren kann ausgewählt sein aus der Gruppe bestehend aus Pulverbettverfahren, Freiraumverfahren und Flüssigmaterialverfahren.

Das Pulverbettverfahren kann ausgewählt sein aus der Gruppe bestehend aus selektives Laserschmelzen, selektives Lasersintern, selektives Hitzesintern (Selective Heat Sintering), Verfestigen von Pulvermaterial mittels Binder (Binder Jetting) und Elektronenstrahlschmelzen.

Das Freiraumverfahren kann ausgewählt sein aus der Gruppe bestehend aus Schmelzschichtung (Fused Deposition Modelling), LOM-Verfahren (Laminated Object Modelling-Verfahren), Auftragsschweißen (Cladding), Wachs-Depositions-Modellierung (Wax Deposition Modelling), Contour Grafting, Kaltgasspritzen und Elektronenstrahlschmelzen.

Das Flüssigmaterialverfahren kann ausgewählt sein aus der Gruppe bestehend aus Stereolithografie, DLP-Verfahren (Digital Light Processing-Verfahren) und LCM-Verfahren. Das LCM-Verfahren kann ein Liquid Composite Moulding-Verfahren oder ein Lithography-based Ceramic Manufacturing-Verfahren sein.

Das medizinische Produkt weist in einer weiteren Ausführungsform ferner ein Abdeckelement auf. Unter dem Ausdruck "Abdeckelement" soll im Sinne der vorliegenden Erfindung ein Element verstanden werden, welches derart ausgestaltet ist, dass es die Abdeckung bzw. den Verschluss einer Knochenkavität ermöglicht. Dadurch eignet sich das medizinische Produkt mit besonderem Vorteil nicht nur zur Auffüllung einer Knochenkavität, sondern auch zur Abdeckung bzw. zum Verschluss einer durch die Glieder des medizinischen Produkts aufgefüllten Knochenkavität.

Das Abdeckelement ist vorzugsweise flächig ausgestaltet. Insbesondere kann das Abdeckelement eine zweidimensionale Struktur besitzen.

Das Abdeckelement kann weiterhin eine Gitterstruktur aufweisen oder in Form einer Gitterstruktur vorliegen.

Bevorzugt handelt es sich bei dem Abdeckelement um ein plattenförmiges, insbesondere gitterplattenförmiges, Abdeckelement. Insbesondere kann das Abdeckelement in Form wenigstens einer Platte, insbesondere wenigstens einer Gitterplatte, vorliegen. Der Ausdruck "wenigstens eine Platte" definiert im Sinne der vorliegenden Erfindung eine Platte oder eine Mehrzahl von Platten, d. h. zwei, drei oder mehr Platten. Diese Definition gilt sinngemäß auch für den Ausdruck "wenigstens eine Gitterplatte". Erfindungsgemäß ist es insbesondere bevorzugt, wenn das Abdeckelement in Form einer Platte, insbesondere einer Gitterplatte, vorliegt.

In einer bevorzugten Ausführungsform ist wenigstens ein Teil der Glieder, insbesondere nur ein Teil der Glieder, mit dem Abdeckelement verbunden. Der wenigstens eine Teil der Glieder kann dabei stoffschlüssig mit dem Abdeckelement verbunden sein. Beispielsweise kann die Verbindung zwischen dem wenigstens einen Teil der Glieder und dem Abdeckelement auf einer Klebe- und/oder Schweißverbindung beruhen. Alternativ können der wenigstens eine Teil der Glieder und das Abdeckelement einstückig bzw. einteilig (monolithisch) ausgebildet sein. Insbesondere können die Glieder an die Abdeckplatte angeformt sein. Eine einstückige Ausbildung oder eine Anformung ist beispielsweise mittels eines additiven bzw. generativen Fertigungsverfahrens möglich. Bezüglich geeigneter additiver bzw. generativer Fertigungsverfahren wird vollständig auf die bisherige Beschreibung Bezug genommen.

Das Abdeckelement weist in einer weiteren Ausführungsform ein Befestigungselement oder mehrere Befestigungselemente auf. Dadurch ist eine Befestigung des Abdeckelements und damit des medizinischen Produkts mit umliegendem Knochengewebe möglich. Das Befestigungselement kann/die Befestigungselemente können beispielsweise schrauben-, nagel- und/oder ankerförmig ausgestaltet sein. Alternativ kann das Abdeckelement eine Aufnahmeeinrichtung, beispielsweise in Form einer Öffnung oder Vertiefung, oder mehrere Aufnahmeeinrichtungen, beispielsweise in Form von Öffnungen oder Vertiefungen, zur Aufnahme eines Befestigungselements oder mehrerer Befestigungselemente aufweisen.

Das Abdeckelement ist in einer weiteren Ausführungsform patientenindividuell bzw. -spezifisch ausbildbar.

Bezüglich geeigneter Materialien, welche das Abdeckelement aufweisen kann oder aus welchen das Abdeckelement bestehen kann, wird zur Vermeidung von unnötigen Wiederholungen vollständig auf die im Rahmen der bisherigen Beschreibung im Zusammenhang mit den Gliedern, Berandungen sowie den optional vorgesehenen zusätzlichen Strukturelementen offenbarten Materialien Bezug genommen. Die insoweit beschriebenen Materialien können auch zur Herstellung des Abdeckelements verwendet werden.

In einer weiteren Ausführungsform weist das Abdeckelement einen Wirkstoff auf. Das Abdeckelement kann insbesondere teilweise oder vollständig mit einem Wirkstoff beschichtet sein. Bezüglich Beispiele für einen geeigneten Wirkstoff sei auf die bisherige Beschreibung Bezug genommen.

Das Abdeckelement weist in einer weiteren Ausführungsform lasttragende Eigenschaften auf. Dadurch kann die mechanische Stabilität des Produkts insgesamt verbessert werden.

Ein medizinisches Produkt mit einem Abdeckelement eignet sich insbesondere zur Behandlung von tumorbedingten Röhrenknochenkavitäten, insbesondere von tumorbedingten Kavitäten des Oberschenkel- und/oder Oberarmknochens.

In einer weiteren Ausführungsform weist das medizinische Produkt ferner ein Einfassungselement (Bordierungselement) auf. Unter dem Ausdruck "Einfassungselement" ("Bordierungselement") soll im Sinne der vorliegenden Erfindung ein Element verstanden werden, welches derart ausgestaltet ist, dass es eine Einfassung der Glieder des medizinischen Produkts bewirken kann.

Das Einfassungselement besitzt vorzugsweise lasttragende Eigenschaften. Dadurch ist insgesamt eine höhere mechanische Stabilität des medizinischen Produkts erzielbar.

Vorzugsweise ist das Einfassungselement flächig ausgestaltet. Insbesondere kann das Einfassungselement eine zweidimensionale Struktur besitzen.

In einer weiteren Ausführungsform weist das Einfassungselement eine Gitterstruktur auf oder liegt in Form einer Gitterstruktur vor.

In einer weiteren Ausführungsform ist das Einfassungselement plattenförmig, insbesondere gitterplattenförmig, ausgebildet. Insbesondere kann das Einfassungselement wenigstens eine Platte, insbesondere wenigstens eine Gitterplatte, aufweisen oder in Form wenigstens einer Platte, insbesondere wenigstens einer Gitterplatte, vorliegen.

In einer weiteren Ausführungsform weist das Einfassungselement wenigstens ein Einfassungsteil, d. h. ein Einfassungsteil (Bordierungsteil) oder eine Mehrzahl von Einfassungsteilen (Bordierungsteilen), auf. Erfindungsgemäß ist es bevorzugt, wenn das Einfassungselement eine Mehrzahl von Einfassungsteilen, d. h. zwei oder mehr Einfassungsteile, insbesondere drei Einfassungsteile, aufweist.

In einer weiteren Ausführungsform weist das Einfassungselement drei Einfassungsteile auf, wobei es sich bei den Einfassungsteilen um ein viereckförmiges, insbesondere rechteckförmiges, Einfassungsteil und zwei dreieckförmige, insbesondere zwei nicht gleichseitig dreieckförmige, bevorzugt zwei rechtwinklig-dreieckförmige, Einfassungsteile handelt. Bevorzugt sind die beiden dreieckförmigen Einfassungsteile jeweils über eine ihrer Katheten mit gegenüberliegenden Seiten, vorzugsweise gegenüberliegenden Schmalseiten, des viereckförmigen Einfassungsteils verbunden. Die dreieckförmigen Einfassungsteile sind vorzugsweise parallel oder im Wesentlichen parallel zueinander angeordnet. Die freien Ecken der dreieckförmigen Einfassungsteile, d. h. die Ecken der dreieckförmigen Einfassungsteile, welche nicht in eine Verbindung mit dem viereckförmigen Einfassungsteil einbezogen sind, weisen vorzugsweise in dieselbe Richtung. Unter Stabilitätsgesichtspunkten kann es von Vorteil sein, wenn die freien Ecken der dreieckförmigen Einfassungsteile über einen Steg miteinander verbunden sind. Im Falle von nicht gleichseitig dreieckförmigen Einfassungsteilen, insbesondere im Falle von rechtwinkligdreieckförmigen Einfassungsteilen, sind diese vorzugsweise jeweils über ihre kürzeren Katheten mit gegenüberliegenden Seiten, vorzugsweise gegenüberliegenden Schmalseiten, des viereckförmigen Einfassungsteils verbunden. Die dreieckförmigen Einfassungsteile sind auch in diesem Fall bevorzugt parallel oder im Wesentlichen parallel zueinander angeordnet. Insbesondere weisen auch in diesem Fall die freien Ecken der dreieckförmigen Einfassungsteile in dieselbe Richtung. In diesem Fall werden die freien Ecken jeweils durch die Hypotenuse sowie die längere Kathete der dreieckförmigen Einfassungsteile gebildet. Auch in diesem Fall kann es unter Stabilitätsgesichtspunkten günstig sein, wenn die freien Ecken der dreieckförmigen Einfassungsteile über einen Steg miteinander verbunden sind.

In einer weiteren Ausführungsform ist wenigstens ein Teil der Glieder, insbesondere nur ein Teil der Glieder, mit dem Einfassungselement, insbesondere an einer Innenseite oder ggf. mehreren Innenseiten des Einfassungselements, verbunden. Der wenigstens eine Teil der Glieder kann dabei stoffschlüssig mit dem Einfassungselement, insbesondere mit einer Innenseite oder ggf. mehreren Innenseiten des Einfassungselements, verbunden sein. Die stoffschlüssige Verbindung kann beispielsweise auf einer Klebe- und/oder Schweißverbindung beruhen. Alternativ können der wenigstens eine Teil der Glieder und das Einfassungselement einstückig bzw. einteilig (monolithisch) ausgebildet sein. Insbesondere können die Glieder an das Einfassungselement angeformt sein. Eine einstückige Ausbildung oder eine Anformung ist insbesondere mittels eines additiven bzw. generativen Fertigungsverfahrens möglich. Bezüglich geeigneter additiver bzw. generativer Fertigungsverfahren wird vollständig auf die bisherige Beschreibung Bezug genommen.

Das Einfassungselement ist in einer weiteren Ausführungsform patientenindividuell bzw. -spezifisch ausbildbar.

Bezüglich geeigneter Materialien, welche das Einfassungselement, insbesondere die zuvor genannten Einfassungsteile, aufweisen kann oder aus welchen das Einfassungselement, insbesondere die zuvor beschriebenen Einfassungsteile, bestehen kann, wird vollständig auf die im Rahmen der bisherigen Beschreibung im Zusammenhang mit den Gliedern, Berandungen sowie den optional vorgesehenen zusätzlichen Strukturelementen offenbarten Materialien Bezug genommen. Die insoweit beschriebenen Materialien können auch zur Herstellung des Einfassungselements, insbesondere der zuvor beschriebenen Einfassungsteile, verwendet werden.

Das Einfassungselement weist in einer weiteren Ausführungsform ein Befestigungselement oder mehrere Befestigungselemente auf. Dadurch ist eine Befestigung des Einfassungselements und damit des medizinischen Produkts mit umliegendem Knochengewebe möglich. Das Befestigungselement kann/die Befestigungselemente können beispielsweise schrauben-, nagel- und/oder ankerförmig ausgestaltet sein. Alternativ kann das Einfassungselement eine Aufnahmeeinrichtung, beispielsweise in Form einer Öffnung oder Vertiefung, oder mehrere Aufnahmeeinrichtungen, beispielsweise in Form von Öffnungen oder Vertiefungen, zur Aufnahme eines Befestigungselements oder mehrerer Befestigungselemente aufweisen.

In einer weiteren Ausführungsform weist das Einfassungselement einen Wirkstoff auf. Insbesondere kann das Einfassungselement teilweise oder vollständig mit einem Wirkstoff beschichtet sein. Bezüglich Beispiele für einen geeigneten Wirkstoff wird auf die bisherige Beschreibung Bezug genommen.

Ein medizinisches Produkt mit einem Einfassungselement eignet sich insbesondere zur Anwendung bei der Durchführung einer totalen Hüftarthroplastik, d. h. bei der Durchführung eines vollständigen Ersatzes eines Hüftgelenks.

Bei dem medizinischen Produkt handelt es sich in einer weiteren Ausführungsform um ein chirurgisches Implantat, vorzugsweise um ein Knochenersatzmaterial, insbesondere zur Unterfütterung von Knochen- oder Gelenkimplantaten oder zur Verwendung als Gegenlager für Knochen- oder Gelenkimplantate.

In einer weiteren Ausführungsform ist das medizinische Produkt zur Anwendung bei der Behandlung, insbesondere zur Anwendung beim Auffüllen und/oder beim Verschluss, einer Knochenkavität, insbesondere einer
- traumabedingten Knochenkavität und/oder
- krankheitsbedingten, insbesondere tumorbedingten, Knochenkavität und/oder
- einer durch eine chirurgische Intervention/Reintervention bedingten Knochenkavität, insbesondere einer nach einer totalen Hüft- oder Kniearthroplastik revisionsbedingten Knochenkavität vorgesehen.

Bei der in der vorherigen Ausführungsform erwähnten Knochenkavität handelt es sich vorzugsweise um eine Gelenksknochenkavität, Röhrenknochenkavität und/oder Wirbelkörperkavität. Besonders bevorzugt handelt es sich bei Knochenkavität um eine Hüftgelenksknochenkavität, Kniegelenksknochenkavität, Oberschenkelknochenkavität, Schienbeinkavität, Wadenbeinkavität, Oberarmknochenkavität, Speichenkavität und/oder Ellenkavität.

Gemäß einem zweiten Aspekt betrifft die Erfindung ein Verfahren zur Herstellung eines medizinischen Produkts gemäß erstem Erfindungsaspekt.

Das Verfahren zeichnet sich dadurch aus, dass eine Mehrzahl von Gliedern, welche jeweils eine umlaufende Berandung aufweisen, mittels eines additiven bzw. generativen Fertigungsverfahrens hergestellt und derart miteinander verbunden werden, dass die Berandungen benachbarter Glieder jeweils ineinandergreifen oder ineinander eingreifen bzw. ineinandergefügt sind.

In einer weiteren Ausführungsform wird ein additives bzw. generatives Fertigungsverfahren verwendet, welches ausgewählt ist aus der Gruppe bestehend aus Pulverbettverfahren, Freiraumverfahren und Flüssigmaterialverfahren. Bezüglich weiterer Merkmale und Vorteile dieser Fertigungsverfahren wird vollständig auf die bereits im Zusammenhang mit dem ersten Erfindungsaspekt beschriebenen Verfahren Bezug genommen.

Vorzugsweise wird als additives bzw. generatives Fertigungsverfahren ein 3D-Druckverfahren verwendet, insbesondere ausgewählt aus der Gruppe bestehend aus selektives Laserschmelzen, Elektrodenstrahlschmelzen, selektives Lasersintern, Stereolithografie, Digital Light Processing, Polyjet-Modeling und Fused Deposition Modeling.

In einer weiteren Ausführungsform werden die Glieder derart miteinander verbunden, dass die Berandungen der Glieder jeweils in eine durchgehende Öffnung, in ein durchgehendes Loch oder in einen durchgehenden Kanal von wenigstens einem benachbarten Glied, insbesondere von zwei, drei oder vier benachbarten Gliedern, eingreifen.

In einer weiteren Ausführungsform werden mittels des additiven bzw. generativen Fertigungsverfahrens ferner Greifelemente ausgebildet und mit manchen der Glieder, insbesondere mit Gliedern, welche für eine Anordnung an Rand- und/oder Kanten- und/oder Eckbereichen des medizinischen Produkts vorgesehen sind, verbunden. Vorzugsweise werden mittels des additiven bzw. generativen Fertigungsverfahrens ferner Greifelemente an manche der Glieder, insbesondere an Glieder, welche für eine Anordnung an Rand- und/oder Kanten- und/oder Eckbereichen des medizinischen Produkts vorgesehen sind, angeformt.

In einer weiteren Ausführungsform werden mittels des additiven bzw. generativen Fertigungsverfahrens ferner zusätzliche Strukturelemente ausgebildet oder erzeugt und zwischen wenigstens manchen der Glieder angeordnet oder integriert, insbesondere in Hohl- und/oder Zwischenräume des medizinischen Produkts, bevorzugt in Hohl- und/oder Zwischenräume einer aus den Gliedern aufgebauten Struktur, angeordnet oder integriert.

In einer weiteren Ausführungsform werden mittels des additiven bzw. generativen Fertigungsverfahrens ferner zusätzliche Strukturelemente mit wenigstens manchen der Glieder verbunden. Vorzugsweise werden mittels des additiven bzw. generativen Fertigungsverfahrens ferner zusätzliche Strukturelemente an wenigstens manche der Glieder angeformt.

In einer weiteren Ausführungsform wird mittels des additiven bzw. generativen Fertigungsverfahrens ferner ein Abdeckelement ausgebildet und mit wenigstens manchen der Glieder verbunden. Vorzugsweise werden wenigstens manche der Glieder mittels des additiven bzw. generativen Fertigungsverfahrens ferner an ein Abdeckelement angeformt.

In einer weiteren Ausführungsform wird mittels des additiven bzw. generativen Fertigungsverfahrens ferner ein Einfassungselement (Bordierungselement) ausgebildet und mit wenigstens manchen der Glieder verbunden. Vorzugsweise werden wenigstens manche der Glieder mittels des additiven bzw. generativen Fertigungsverfahrens ferner an ein Einfassungselement (Bordierungselement) angeformt.

In einer weiteren Ausführungsform werden die Glieder oder Berandungen und/oder optional vorhandene Greifelemente und/oder optional vorhandene zusätzliche Strukturelemente und/oder ein optional vorhandenes Abdeckelement und/oder ein optional vorhandenes Einfassungselement mittels des additiven bzw. generativen Fertigungsverfahrens ferner mit einem Wirkstoff versehen, vorzugsweise beschichtet.

Bezüglich weiterer Merkmale und Vorteile des Verfahrens sowie des medizinischen Produkts wird vollständig auf die im Rahmen des ersten Erfindungsaspekts gemachten Ausführungen Bezug genommen.

Weitere Vorteile und Merkmale der Erfindung ergeben sich aus den Ansprüchen sowie aus der nachfolgenden Beschreibung bevorzugter Ausführungsbeispiele der Erfindung, die anhand der Figuren dargestellt sind.

### KURZBESCHREIBUNGEN DER FIGUREN

In den Figuren ist schematisch Folgendes gezeigt:
- Fig. 1a-c:: Ausschnitte aus verschiedenen Ausführungsformen eines erfindungsgemäßen Produkts,
- Fig. 2a-c:: verschiedene Ausführungsformen eines nicht erfindungsgemäßen Produkts,
- Fig. 3: eine weitere Ausführungsform eines erfindungsgemäßen Produkts,
- Fig. 4: eine weitere Ausführungsform eines erfindungsgemäßen Produkts und
- Fig. 5a-c:: weitere Ausführungsformen eines erfindungsgemäßen Produkts.

### AUSFÜHRLICHE FIGURENBESCHREIBUNG

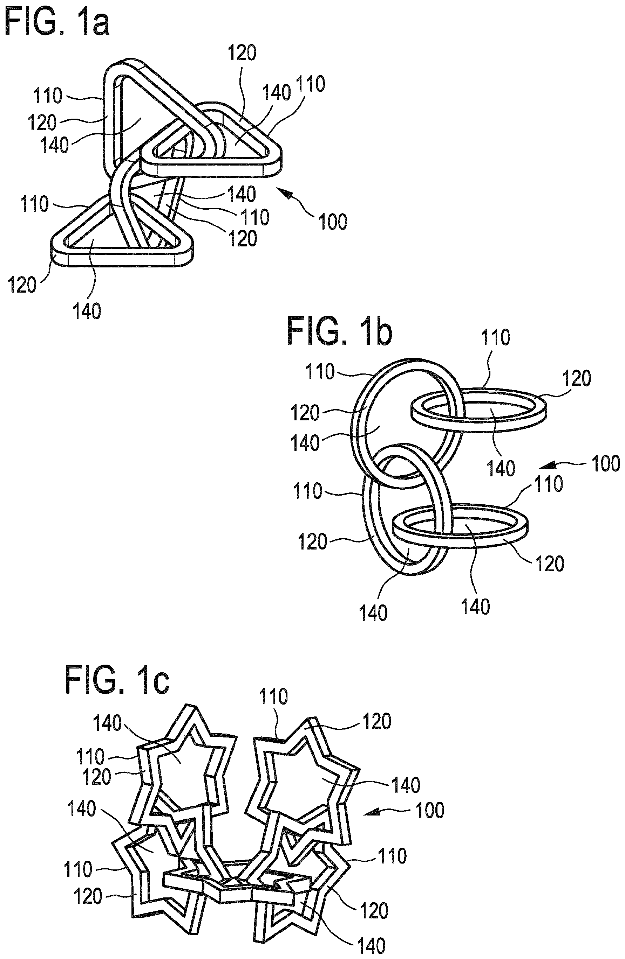

Fig. 1a zeigt einen Ausschnitt aus einem erfindungsgemäßen medizinischen Produkt 100. Das Produkt 100 weist dreieckig ausgestaltete Glieder 110 mit einer umlaufenden Berandung 120 auf. Die Ecken der Glieder 110 sind vorzugsweise abgerundet ausgebildet. Die Berandungen 120 sind jeweils geschlossen, d.h. ohne Unterbrechung, ausgebildet. Weiterhin definieren die Berandungen 120 jeweils eine durchgehende Öffnung 140.

Die Glieder 110 sind miteinander verbunden, wobei die Verbindung darauf beruht, dass die Berandungen 120 benachbarter Glieder 110 ineinandergreifen bzw. ineinandergefügt sind.

Bei dem in Fig. 1a dargestellten Ausschnitt greifen die Berandungen der zwei inneren Glieder jeweils in die Berandungen der unmittelbar benachbarten Glieder ein, während die Berandungen der beiden äußeren Glieder jeweils nur in die Berandung des unmittelbar benachbarten inneren Glieds eingreifen.

Fig. 1b zeigt einen Ausschnitt aus einem weiteren erfindungsgemäßen Produkt 100. Das Produkt 100 weist ringförmig ausgestaltete Glieder 110 mit einer umlaufenden Berandung 120 auf. Die Berandungen 120 sind jeweils geschlossen, d.h. ohne Unterbrechung, ausgebildet. Jede Berandung 120 definiert eine durchgehende Öffnung 140.

Im Übrigen gelten die Ausführungen zu dem in Fig. 1a dargestellten erfindungsgemäßen Produkt 100 sinngemäß.

Fig. 1c zeigt einen Ausschnitt aus einem weiteren erfindungsgemäßen Produkt 100. Das Produkt 100 weist sternförmig ausgestaltete Glieder 110 mit einer umlaufenden Berandung 120 auf. Die Ecken der Glieder 110 sind vorzugsweise nicht abgerundet ausgebildet. Die Berandungen 120 sind jeweils geschlossen, d.h. ohne Unterbrechung, ausgebildet. Jede Berandung 120 definiert eine durchgehende Öffnung 140.

Im Übrigen gelten die Ausführungen zu dem in Fig. 1a dargestellten erfindungsgemäßen Produkt 100 sinngemäß.

Bei den in den Fig. 1a bis 1c dargestellten Ausschnitten eines erfindungsgemäßen Produktes 100 kommt es aufgrund des gegenseitigen Ineinandergreifens von benachbarten Gliederberandungen im Ergebnis zu einer Verkettung der Glieder 110.

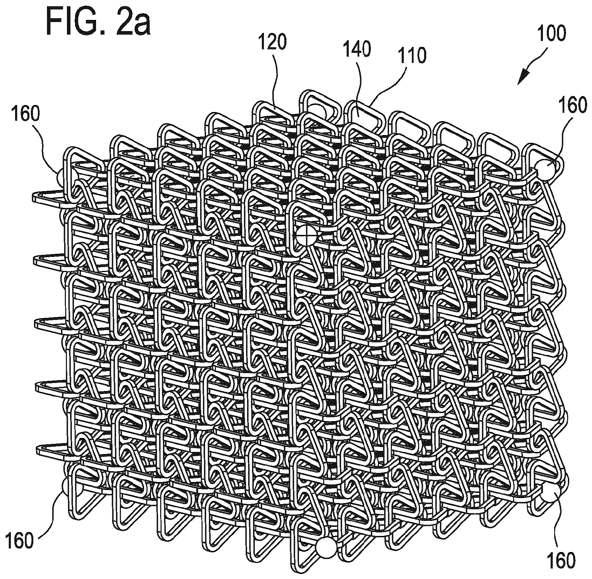

Fig. 2a zeigt eine Ausführungsform eines nicht erfindungsgemäßen medizinischen Produkts 100. Das medizinische Produkt 100 weist eine Vielzahl von Gliedern 110 auf. Die Glieder 110 sind dreieckig ausgestaltet, wobei die Ecken der Glieder 110 vorzugsweise abgerundet sind. Jedes Glied 110 besitzt eine umlaufende und vorzugsweise geschlossene, d.h. ununterbrochene, Berandung 120. Die Berandungen 120 umschließen jeweils eine Öffnung 140. Die Glieder 110 sind miteinander verbunden, wobei die Verbindung der Glieder 110 darauf beruht, dass die Berandungen 120 benachbarter Glieder 110 ineinandergreifen bzw. ineinandergefügt sind. Bevorzugt greifen die Berandungen 120 benachbarter Glieder 110 derart ineinander, dass die verbundenen Glieder 110 relativ zueinander begrenzt beweglich sind.

Das medizinische Produkt 100 weist eine Mehrzahl von innen liegenden Gliedern auf, welche jeweils mit einer Mehrzahl von weiteren Gliedern, insbesondere vier weiteren Gliedern, verbunden sind, wobei das Produkt 100 außenseitig von einer Anzahl von Bereichen mit jeweils entlang seiner Oberfläche miteinander verbundenen Gliedern begrenzt wird.

Die Glieder 110 sind relativ zueinander begrenzt beweglich. Dies erlaubt mit besonderem Vorteil eine Kompression des medizinischen Produkts 100, um beispielsweise in Knochenkavitäten eingeführt zu werden. Die begrenzte Beweglichkeit der Glieder 110 erlaubt umgekehrt auch eine Expansion bzw. Entfaltung des Produkts, beispielsweise in eine würfelförmige Struktur, wie in Fig. 2a dargestellt. Die Expansion bzw. Entfaltung des medizinischen Produkts 100 kann durch Greif- oder Halteelemente 160 erleichtert werden. Die Greif- bzw. Halteelemente 160 können beispielsweise, wie in Fig. 2a dargestellt, kugelförmig ausgestaltet sein. Zweckmäßigerweise befinden sich die Greif- bzw. Halteelemente 160 nach Expansion bzw. Entfaltung des Produkts 100 an den Ecken des expandierten bzw. entfalteten Produkts.

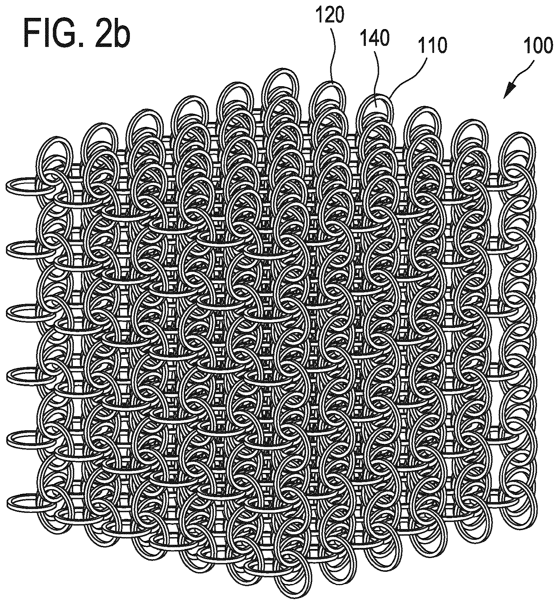

Fig. 2b zeigt eine weitere Ausführungsform eines nicht erfindungsgemäßen medizinischen Produkts 100. Das medizinische Produkt 100 weist eine Vielzahl von Gliedern 110 auf. Die Glieder 110 sind ringförmig ausgestaltet. Jedes Glied 110 besitzt eine umlaufende und vorzugsweise geschlossene, d.h. ununterbrochene, Berandung 120. Die Berandungen 120 umschließen jeweils eine Öffnung 140. Die Glieder 110 sind miteinander verbunden, wobei die Verbindung der Glieder 110 darauf beruht, dass die Berandungen 120 benachbarter Glieder 110 ineinandergreifen bzw. ineinandergefügt sind. Bevorzugt greifen die Berandungen 120 benachbarter Glieder 110 derart ineinander, dass die verbundenen Glieder 110 relativ zueinander begrenzt beweglich sind. Im Übrigen gelten die Ausführungen zu dem in Fig. 2a dargestellten nicht erfindungsgemäßen Produkt 100 sinngemäß.

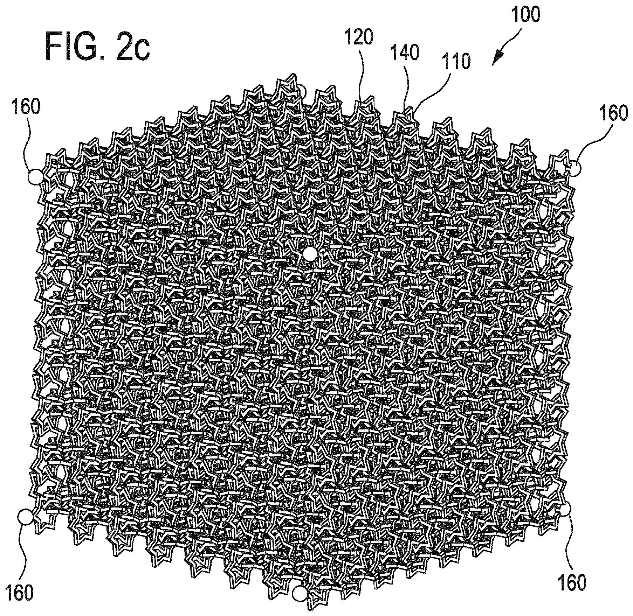

Fig. 2c zeigt eine weitere Ausführungsform eines nicht erfindungsgemäßen medizinischen Produkts 100. Das medizinische Produkt 100 weist eine Vielzahl von Gliedern 110 auf. Die Glieder 110 sind sternförmig ausgestaltet. Jedes Glied 110 besitzt eine umlaufende und vorzugsweise geschlossene, d.h. ununterbrochene, Berandung 120. Die Berandungen 120 umschließen jeweils eine Öffnung 140. Die Glieder 110 sind miteinander verbunden, wobei die Verbindung der Glieder 110 darauf beruht, dass die Berandungen 120 benachbarter Glieder 110 ineinandergreifen bzw. ineinandergefügt sind. Die Berandungen 120 benachbarter Glieder 110 greifen bevorzugt derart ineinander, dass die verbundenen Glieder 110 relativ zueinander begrenzt beweglich sind. Die Expansion bzw. Entfaltung des medizinischen Produkts 100 kann durch Greif- oder Halteelemente 160 erleichtert werden. Die Greif- bzw. Halteelemente 160 können beispielsweise, wie in Fig. 2c dargestellt, kugelförmig ausgestaltet sein. Zweckmäßigerweise befinden sich die Greif- bzw. Halteelemente 160 nach Expansion bzw. Entfaltung des Produkts 100 an den Ecken des expandierten bzw. entfalteten Produkts.

Im Übrigen gelten die Ausführungen zu dem in Fig. 2a dargestellten nicht erfindungsgemäßen Produkt 100 sinngemäß.

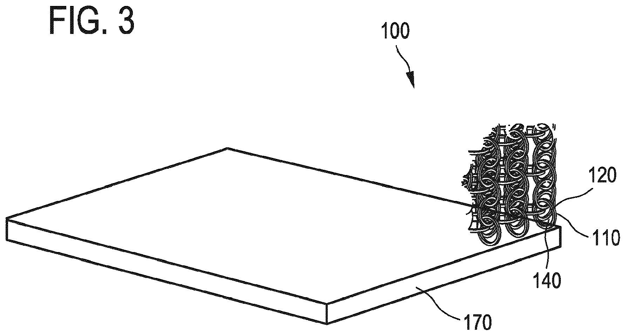

Fig. 3 zeigt eine weitere Ausführungsform eines erfindungsgemäßen medizinischen Produkts 100. Das medizinische Produkt 100 weist eine Vielzahl von Gliedern 110 (ausschnittsweise dargestellt) sowie ein Abdeckelement 170 auf.

Das Abdeckelement 170 ist vorzugsweise plattenförmig ausgebildet.

Die Glieder 110 können - wie dargestellt - beispielsweise ringförmig ausgestaltet sein. Jedes Glied 110 besitzt eine umlaufende, vorzugsweise geschlossene, d. h. ununterbrochene, Berandung 120. Die Berandungen 120 umschließen jeweils eine Öffnung 140. Die Glieder 110 sind miteinander verbunden, wobei die Verbindung der Glieder 110 darauf beruht, dass die Berandungen 120 benachbarter Glieder 110 ineinandergreifen bzw. ineinandergefügt sind.

Ein Teil der Glieder 110 ist mit dem Abdeckelement 170 verbunden.

Das medizinische Produkt gemäß Fig. 3 eignet sich insbesondere zur Anwendung beim Auffüllen und Verschluss einer Knochenkavität. Ein Auffüllen der Knochenkavität kann dabei mit besonderem Vorteil mittels der Glieder 110 bzw. einer durch die Glieder 110 aufgebauten Struktur vorgenommen werden, während eine Abdeckung oder ein Verschluss der Knochenkavität über das Abdeckelement 170 erfolgen kann.

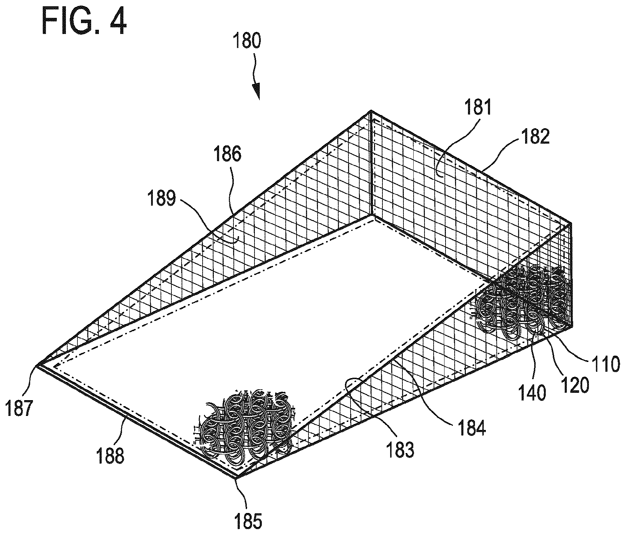

Fig. 4 zeigt eine weitere Ausführungsform eines erfindungsgemäßen medizinischen Produkts 100. Das medizinische Produkt 100 weist eine Vielzahl von Gliedern 110 (ausschnittsweise dargestellt) sowie ein Einfassungselement 180 auf.

Das Einfassungselement 180 weist ein rechteckförmiges Einfassungsteil 182 sowie zwei nicht gleichseitig dreieckförmige, insbesondere zwei rechtwinklig-dreieckförmige, Einfassungsteile 184; 186 auf. Die dreieckförmigen Einfassungsteile 184; 186 sind jeweils über ihre kürzere Kathete mit einer gegenüberliegenden Schmalseite des rechteckförmigen Einfassungsteils 182 verbunden. Die freien Ecken 185; 187 der Einfassungsteile 184; 186 weisen jeweils in dieselbe Richtung. Die freien Ecken 185; 187 werden jeweils durch die Hypotenuse sowie die längere Kathete der Einfassungselemente 184; 186 gebildet. Die Einfassungelemente 184; 186 sind weiterhin vorzugsweise parallel oder im Wesentlichen parallel zueinander angeordnet. Weiterhin sind die freien Ecken 185; 187 vorzugsweise über einen Steg 188 miteinander verbunden. Dadurch lässt sich die mechanische Stabilität des Einfassungselements 180 und damit insgesamt des medizinischen Produkts 100 erhöhen.

Die Einfassungselemente 182; 184; 186 sind vorzugsweise plattenförmig ausgestaltet.

Die Glieder 110 können - wie dargestellt - beispielsweise ringförmig ausgestaltet sein. Jedes Glied 110 besitzt eine umlaufende und vorzugsweise geschlossene, d. h. ununterbrochene, Berandung 120. Die Berandungen 120 umschließen jeweils eine Öffnung 140. Die Glieder 110 sind miteinander verbunden, wobei die Verbindung der Glieder 110 darauf beruht, dass die Berandungen 120 benachbarter Glieder 110 ineinandergreifen bzw. ineinandergefügt sind.

Ein Teil der Glieder 110 ist mit einer Innenfläche des Einfassungselements 180 verbunden.

Unter dem Ausdruck "Innenfläche des Einfassungselements" soll im Sinne der vorliegenden Erfindung die Fläche des Einfassungselements verstanden werden, welche einem von dem Einfassungselement eingefassten Bereich direkt zugewandt ist. Anders ausgedrückt, handelt es sich bei der Innenfläche des Einfassungselements um die Fläche, welche einen von dem Einfassungselement eingefassten Bereich unmittelbar umgibt.

Bei dem in Fig. 4 dargestellten Produkt 100 kann ein Teil der Glieder 110 beispielsweise mit der Innenfläche 181 des rechteckförmigen Einfassungsteils 182 und/oder der Innenfläche 183 des dreieckförmigen Einfassungsteils 184 und/oder der Innenfläche 189 des dreieckförmigen Einfassungsteils 186 verbunden sein. Dargestellt ist eine Befestigung der Glieder 110 an der Innenfläche 181 des Einfassungsteils 182 sowie an der Innenfläche 183 des Einfassungsteils 184.

Das medizinische Produkt gemäß Fig. 4 eignet sich insbesondere zur Anwendung bei einer totalen Hüftarthroplastik.

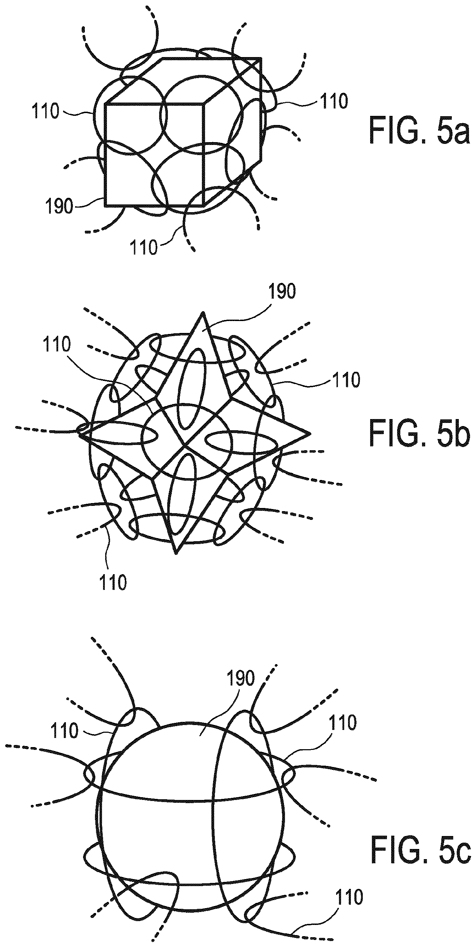

Figur 5a zeigt ausschnittsweise eine weitere Ausführungsform eines medizinischen Produkts 100. Das medizinische Produkt 100 weist eine Vielzahl von ringförmig ausgestalteten Gliedern 110 auf. Jedes Glied 110 besitzt eine umlaufende und vorzugsweise geschlossene Berandung.

Die Glieder 110 sind derart miteinander verbunden, dass die Berandungen benachbarter Glieder 110 ineinander eingreifen.

Das medizinische Produkt 100 weist ferner zusätzliche Strukturelemente auf, welche würfelförmig ausgebildet und im Inneren des medizinischen Produkts 100, insbesondere in Hohl- und/oder Zwischenräumen des medizinischen Produkts 100, angeordnet oder integriert sind.

In der ausschnittsweisen Darstellung von Figur 5a ist ein solches zusätzliches Strukturelement 190 gezeigt. Das würfelförmige Strukturelement 190 ist größer ausgebildet als die Glieder 110. Das würfelförmige Strukturelement 190 ist in einem Zwischenraum angeordnet oder integriert, welcher von miteinander verbundenen Gliedern 110 gebildet wird. Vorzugsweise ragt jede Ecke der würfelförmigen Strukturelemente - wie am Beispiel des dargestellten würfelförmigen Strukturelements 190 gezeigt - durch ein ringförmig ausgestaltetes Glied 110 hindurch. Auf diese Weise ist eine Stabilisierung/Aussteifung des medizinischen Produkts erzielbar. Gleichzeitig kann eine unbeabsichtigte Freisetzung der zusätzlichen Strukturelemente aus dem medizinischen Produkt 100 verhindert werden.

Figur 5b zeigt ausschnittsweise eine weitere Ausführungsform eines medizinischen Produkts 100. Das medizinische Produkt 100 weist eine Vielzahl von ringförmig ausgebildeten Gliedern 110 sowie zusätzliche Strukturelemente in Form von vierzackigen Sternen auf. Die zusätzlichen Strukturelemente sind im Inneren des medizinischen Produkts 100, insbesondere in Hohl- und/oder Zwischenräumen des medizinischen Produkts 100, angeordnet oder integriert.

Bei der ausschnittsweisen Darstellung von Figur 5b ist ein derartiges zusätzliches Strukturelement 190 dargestellt. Das sternenförmige Strukturelement 190 ist größer ausgebildet als die Glieder 110. Das sternenförmige Strukturelement 190 ist in einem Zwischenraum angeordnet oder integriert, welcher von miteinander verbundenen Gliedern 110 gebildet wird. Vorzugsweise ragt jede Zacke der sternenförmigen Strukturelemente - wie am Beispiel des dargestellten sternenförmigen Strukturelements 190 gezeigt - durch ein ringförmiges Glied 110 hindurch. Auf diese Weise ist ebenfalls eine Stabilisierung/Aussteifung des medizinischen Produkts 100 erzielbar, ohne dass es zu einer unbeabsichtigten Freisetzung der zusätzlichen Strukturelemente aus dem medizinischen Produkt 100 kommt.

Bezüglich weiterer Merkmale und Vorteile des medizinischen Produkts 100 wird auf die Ausführungen zur Fig. 5a Bezug genommen.

Figur 5c zeigt ausschnittsweise eine weitere Ausführungsform eines erfindungsgemäßen medizinischen Produkts 100. Das medizinische Produkt 100 weist eine Vielzahl von ringförmig ausgestalteten Gliedern 110 sowie zusätzliche Strukturelemente in Form von Kugeln auf. Die zusätzlichen Strukturelemente sind im Inneren des medizinischen Produkts 100, insbesondere in Hohl- und/oder Zwischenräumen des medizinischen Produkts 100, angeordnet oder integriert.

Bei der ausschnittsweisen Darstellung der Figur 5c ist ein derartiges Strukturelement 190 gezeigt. Das kugelförmige Strukturelement 190 ist größer ausgebildet als die Glieder 110. Das kugelförmige Strukturelement 190 ist in einem Zwischenraum angeordnet oder integriert, welcher von miteinander verbundenen Gliedern 110 gebildet wird. Vorzugsweise ragen Teile der Außenoberfläche der kugelförmigen Strukturelemente - wie am Beispiel des dargestellten kugelförmigen Strukturelements 190 gezeigt - jeweils durch ein ringförmiges Glied 110 hindurch. Dadurch kann ebenfalls eine Stabilisierung/Aussteifung des medizinischen Produkts 100 realisiert werden, ohne dass eine unbeabsichtigte Freisetzung der zusätzlichen Strukturelemente aus dem medizinischen Produkt 100 zu befürchten ist.

Bezüglich weiterer Merkmale und Vorteile des medizinischen Produkts 100 wird auf die Ausführungen zur Fig. 5a Bezug genommen.

Bei den in den Figuren 1 bis 5 dargestellten Ausführungsformen handelt es sich jeweils bevorzugt um ein chirurgisches Implantat, vorzugsweise um ein Knochenersatzmaterial, insbesondere zur Unterfütterung von Knochen- oder Gelenkimplantaten oder zur Verwendung als Gegenlager für Knochen- oder Gelenkimplantate.

### AUSFÜHRUNGSBEISPIEL (nicht erfindunasaemäß)

Mittels CAD (computer-aided design, rechnerunterstütztes Konstruieren) wurde ein medizinisches Produkt in Form eines chirurgischen Implantats, welches sich insbesondere zur Unterfütterung von Knochen- oder Gelenkimplantaten oder zur Verwendung als Gegenlager für Knochen- oder Gelenkimplantate eignet, konstruiert. Das konstruierte Implantat wies eine Mehrzahl von miteinander verbundenen, ringförmig ausgestalteten Gliedern auf. Dabei wurde das chirurgische Implantat derart konstruiert, dass die Berandungen benachbarter Glieder ineinander griffen, bzw. ineinander gefügt waren, so dass die verbundenen Glieder begrenzt relativ zueinander beweglich sind.

Die CAD-Daten wurden anschließend als STL-Datei (STereoLithography Standard Tessellation Language) exportiert und in eine 3D Drucker-Software importiert. Dort wurden die STL-Daten für den Druckvorgang aufbereitet. Anschließend erfolgte die Herstellung des chirurgischen Implantats mittels 3D Druck. Nach Beendigung der Herstellung wurde das chirurgische Implantat aus dem 3D Drucker entnommen und von Rückständen gesäubert. Abhängig vom Druckergebnis konnte in Einzelfällen eine Nachbearbeitung, insbesondere ein Optimieren und/oder Re-Designen der CAD-Daten und/oder STL-Daten, erforderlich sein. Danach wurde das Implantat gereinigt sowie einer Qualitätssicherung unterworfen. Anschließend wurde das chirurgische Implantat verpackt und die Verpackung beschriftet.

Es versteht sich, dass die oben beispielhaft beschriebene Prozesskette für die Herstellung eines erfindungsgemäßen Produkts zusätzliche Verfahrensschritte umfassen kann. Abhängig von der beabsichtigten Funktion des Produkts kann das Herstellungsverfahren zusätzlich einen Beschichtungsschritt sowie ggf. einen sich daran anschließenden Reinigungsschritt umfassen. Des Weiteren kann vor der Verpackung des Produkts eine Sterilisation durchgeführt werden. Schließlich kann nach der Verpackung des Produkts sowie nach einer Beschriftung der Verpackung eine erneute Qualitätssicherung durchgeführt werden.

## Patentansprüche

1. Medizinisches Produkt (100), vorzugsweise zur Anwendung bei der Behandlung, insbesondere beim Auffüllen und/oder Verschluss, einer Knochenkavität, wobei das Produkt (100) eine Mehrzahl von miteinander verbundenen Gliedern (110) aufweist, wobei jedes Glied (110) eine umlaufende Berandung (120) aufweist und die Berandungen (120) benachbarter Glieder (110) ineinandergreifen, wobei das Produkt (100) eine aus den Glieder (110) aufgebaute dreidimensionale Struktur (130) aufweist, wobei die dreidimensionale Struktur (130) einen mehrlagigen Aufbau besitzt, wobei jede Lage miteinander verbundene Glieder (110) aufweist, wobei die Berandungen (120) von Gliedern (110) benachbarter Lagen ineinander greifen, **dadurch gekennzeichnet, dass** ein Teil der Glieder derart miteinander verbunden ist, dass sie nicht relativ zueinander beweglich sind, wobei die Glieder einteilig bzw. monolithisch ausgestaltet und mittels eines additiven bzw. generativen Fertigungsverfahrens hergestellt sind.

2. Medizinisches Produkt (100) nach Anspruch 1, **dadurch gekennzeichnet, dass** eine Mehrzahl von innenliegenden Gliedern (110) mit jeweils einer Mehrzahl von weiteren Gliedern (110) verbunden ist, und dass das Produkt (100) außenseitig von einer Anzahl von Bereichen mit jeweils entlang seiner Oberfläche miteinander verbundenen Gliedern (110) begrenzt wird.

3. Medizinisches Produkt (100) nach Anspruch 1 oder 2, **dadurch gekennzeichnet, dass** es sich bei der Struktur (130) um eine polyederförmige Struktur, insbesondere um eine würfelförmige, quaderförmige, prismenförmige, pyramidenförmige, spatenförmige oder eine aus Freiformflächen begrenzte Struktur, handelt.

4. Medizinisches Produkt (100) nach einem der vorhergehenden Ansprüche, **dadurch gekennzeichnet, dass** es sich bei der Struktur (130) um eine nicht polyederförmige Struktur, insbesondere um eine kugelförmige, ellipsoide oder kegelförmige Struktur, handelt.

5. Medizinisches Produkt (100) nach einem der vorhergehenden Ansprüche, **dadurch gekennzeichnet, dass** wenigstens ein Teil der Berandungen (120) geschlossen ist, wenigstens eine Öffnung bzw. Unterbrechung, insbesondere wenigstens einen Spalt, oder wenigstens eine Sollbruchstelle aufweist.

6. Medizinisches Produkt (100) nach einem der vorhergehenden Ansprüche, **dadurch gekennzeichnet, dass** die Glieder (110) oder die Berandungen (120) dreieckig, viereckig, fünfeckig, sechseckig, ellipsoid, ringförmig, toroidförmig, würfelförmig und/oder sternförmig ausgebildet sind oder eine aus Freiformflächen erstellte Geometrie oder Form besitzen.

7. Medizinisches Produkt (100) nach einem der vorhergehenden Ansprüche, **dadurch gekennzeichnet, dass** die Glieder (110) in zwei oder mehr Gruppen einteilbar sind, wobei die Glieder (110) jeder Gruppe identisch zueinander ausgebildet sind und die Glieder (110) unterschiedlicher Gruppen unterschiedlich zueinander ausgebildet sind, wobei vorzugsweise sich die Glieder (110) unterschiedlicher Gruppen hinsichtlich wenigstens einer Eigenschaft unterscheiden, welche ausgewählt ist aus der Gruppe bestehend aus Gesamtdurchmesser, lichter Durchmesser, Gliedergröße, Gliederform, Gliedermaterial, Berandungsstärke bzw. -dicke, Farbe, Additivierung wie Wirkstoffadditivierung und Kombinationen aus zwei oder mehreren der genannten Eigenschaften.

8. Medizinisches Produkt (100) nach einem der vorhergehenden Ansprüche, **dadurch gekennzeichnet, dass** manche Glieder (110), insbesondere Glieder (110) an Rand-, Kanten- und/oder Eckbereichen des Produkts (100), einen größeren lichten Durchmesser aufweisen als andere Glieder (110) des Produkts (100), insbesondere innere Glieder (110) des Produkts.

9. Medizinisches Produkt (100) nach einem der vorhergehenden Ansprüche, **dadurch gekennzeichnet, dass** manche Glieder (110), insbesondere Glieder (110) an Rand-, Kanten- und/oder Eckbereichen des Produkts (100), eine Markierung, insbesondere farbige Markung, und/oder manche Glieder (110), insbesondere Glieder (110) an Rand-, Kanten- und/oder Eckbereichen des medizinischen Produkts (100), ein Greifelement aufweisen und/oder manche Glieder (110), insbesondere an Rand-, Kanten- und/oder Eckbereichen des Produkts (100), derart miteinander verbunden sind, dass sie nicht relativ zueinander beweglich sind.

10. Medizinisches Produkt (100) nach einem der vorhergehenden Ansprüche, **dadurch gekennzeichnet, dass** die Glieder (110) ein Material aufweisen oder aus einem Material bestehen, welches ausgewählt ist aus der Gruppe bestehend aus Metalle, Polymere, Keramikmaterialien, Knochenzementmaterialien und Mischungen bzw. Kombinationen aus zwei oder mehreren der genannten Materialien.

11. Medizinisches Produkt (100) nach einem der vorhergehenden Ansprüche, **dadurch gekennzeichnet, dass** das medizinische Produkt (100) neben den Gliedern (110) zusätzliche Strukturelemente (190) aufweist.

12. Medizinisches Produkt (100) nach Anspruch 11, **dadurch gekennzeichnet, dass** sich die zusätzlichen Strukturelemente (190) in Hohl- und/oder Zwischenräumen des medizinischen Produkts (100) befinden.

13. Medizinisches Produkt (100) nach einem der vorhergehenden Ansprüche, **dadurch gekennzeichnet, dass** es sich bei dem Produkt (100) um ein chirurgisches Implantat, vorzugsweise um ein Knochenersatzmaterial, handelt.

14. Verfahren zur Herstellung eines medizinischen Produkts (100) nach einem der vorhergehenden Ansprüche, **dadurch gekennzeichnet, dass** eine Mehrzahl von Gliedern (110) mit einer umlaufenden Berandung (120) mittels eines additiven bzw. generativen Fertigungsverfahrens hergestellt und derart miteinander verbunden werden, dass die Berandungen (120) der verbunden Glieder (110) ineinandergreifen.

## Claims

1. Medical product (100), preferably for use in treating, in particular filling and/or closing a bone cavity, wherein the product (100) comprises a plurality of interconnected members (110), wherein each member (110) has a peripheral boundary (120) and the boundaries (120) of adjacent members (110) interlock, wherein the product (100) comprises a three-dimensional structure (130) composed of the members (110), wherein the three-dimensional structure (130) has a multilayer construction, wherein each layer has interconnected members (110), wherein the boundaries (120) of members (110) engage with adjacent layers,
**characterized in that**
a part of the members are interconnected such that they are not moveable relative to one another, wherein the members have a single-part or monolithic configuration and are produced by means of an additive or generative production method.

2. Medical product (100) according to claim 1, **characterized in that** each of a plurality of inner members (110) is connected to a plurality of further members (110), and **in that** the product (100) is externally bounded by a number of areas, each having interlocking members (110) along its surface.

3. Medical product (100) according to claim 1 or 2, **characterized in that** the structure (130) is a polyhedral structure, in particular a cubical, cuboid, prismatic, pyramidal, spade-shaped structure or a structure delimited from free-form surfaces.

4. Medical product (100) according to one of the preceding claims, **characterized in that** the structure (130) is a non-polyhedral structure, in particular a spherical, ellipsoid or conical structure.

5. Medical product (100) according to one of the preceding claims, **characterized in that** at least a part of the boundaries (120) is closed, has at least one opening or interruption, in particular at least one gap, or at least one predetermined breaking point.

6. Medical product (100) according to one of the preceding claims, **characterized in that** the members (110) or the boundaries (120) have a triangular, quadrangular, pentagonal, hexagonal, ellipsoid, ring-shaped, toroidal, cubical and/or star-shaped configuration, or have a geometry or shape created from free-form surfaces.

7. Medical product (100) according to one of the preceding claims, **characterized in that** the members (110) can be divided into two or more groups, wherein the members (110) of each group are identically configured and the members (110) of different groups are differently configured, wherein preferably the members (110) of different groups differ in at least one property that is selected from the group composed of total diameter, inner diameter, member size, member shape, member material, boundary width or thickness, color, additives such as active ingredient additives, and combinations of two or more of said properties.

8. Medical product (100) according to one of the preceding claims, **characterized in that** some members (110), in particular members (110) in rim, edge and/or corner areas of the product (100), have a larger internal diameter than other members (110) of the product (100), in particular inner members (110) of the product.

9. Medical product (100) according to one of the preceding claims, **characterized in that** some members (110), in particular members (110) in rim, edge and/or corner areas of the product (100), have a marking, in particular a colored marking, and/or some members (110), in particular members (110) in rim, edge and/or corner areas of the medical product (100), comprise a gripping element, and/or some members (110), in particular members (110) in rim, edge and/or corner areas of the product (100), are interconnected such that they are not moveable relative to one another.

10. Medical product (100) according to one of the preceding claims, **characterized in that** the members (110) comprise a material or are composed of a material that is selected from the group comprising metals, polymers, ceramic materials, bone cement materials, and mixtures or combinations of two or more of said materials.

11. Medical product (100) according to one of the preceding claims, **characterized in that** the medical product (100) comprises additional structural elements (190) besides the members (110).

12. Medical product (100) according to claim 11, **characterized in that** the additional structural elements (190) are located in hollow spaces and/or interstices of the medical product (100).

13. Medical product (100) according to one of the preceding claims, **characterized in that** the product (100) is a surgical implant, preferably a bone replacement material.

14. Method for producing a medical product (100) according to one of the preceding claims, **characterized in that** a plurality of members (110) is produced with a peripheral boundary (120) by means of an additive or generative production method and interconnected such that the boundaries (120) of the connected members (110) engage with one another.

## Revendications

1. Produit médical (100), de préférence pour l'utilisation dans le traitement, en particulier le remplissage et/ou la clôture, d'une cavité osseuse, le produit (100) présentant une pluralité d'organes (110) connectés les uns aux autres, chaque organe (110) présentant une bordure périphérique (120) et les bordures (120) d'organes adjacents (110) s'engageant les unes dans les autres, le produit (100) présentant une structure tridimensionnelle (130) constituée des organes (110), la structure tridimensionnelle (130) possédant une construction à plusieurs couches, chaque couche présentant des organes connectés les uns aux autres (110), les bordures (120) d'organes (110) de couches adjacentes s'engageant les unes dans les autres, **caractérisé en ce qu'**une partie des organes sont connectés les uns aux autres de telle sorte qu'ils ne puissent pas bouger les uns par rapport aux autres, les organes étant réalisés d'une seule pièce ou sous forme monolithique et étant fabriqués au moyen d'un procédé de fabrication additif ou génératif.

2. Produit médical (100) selon la revendication 1, **caractérisé en ce qu'**une pluralité d'organes (110) situés à l'intérieur sont à chaque fois connectés à une pluralité d'autres organes (110), et **en ce que** le produit (100) est limité du côté extérieur par un certain nombre de régions ayant à chaque fois des organes (110) connectés les uns aux autres le long de sa surface.

3. Produit médical (100) selon la revendication 1 ou 2, **caractérisé en ce que** la structure (130) est une structure polyédrique, en particulier une structure cubique, parallélépipédique, en forme de prisme, en forme de pyramide, en forme de spatule ou une structure limitée constituée de surfaces de formes libres.

4. Produit médical (100) selon l'une quelconque des revendications précédentes, **caractérisé en ce que** la structure (130) est une structure non polyédrique, en particulier une structure sphérique, ellipsoïde ou conique.

5. Produit médical (100) selon l'une quelconque des revendications précédentes, **caractérisé en ce qu'**au moins une partie des bordures (120) est fermée, présente au moins une ouverture ou une interruption, en particulier au moins une fente ou au moins une zone destinée à la rupture.

6. Produit médical (100) selon l'une quelconque des revendications précédentes, **caractérisé en ce que** les organes (110) ou les bordures (120) sont réalisés sous forme triangulaire, quadratique, pentagonale, hexagonale, ellipsoïde, annulaire, toroïdale, cubique et/ou stellaire ou possèdent une géométrie ou une forme constituée à partir de surfaces de formes libres.

7. Produit médical (100) selon l'une quelconque des revendications précédentes, **caractérisé en ce que** les organes (110) peuvent être divisés en deux ou plus de deux groupes, les organes (110) de chaque groupe étant réalisés de manière identique les uns des autres et les organes (110) de groupes différents étant réalisés de manière différente les uns des autres, les organes (110) de groupes différents se distinguant de préférence en termes d'au moins une propriété choisie parmi le groupe constitué du diamètre total, du diamètre intérieur, de la taille des organes, de la forme des organes, du matériau des organes, de la largeur ou de l'épaisseur des bordures, de la couleur, de l'additivation, comme par exemple l'additivation de substances actives et de combinaisons de deux ou plus de deux des propriétés citées.

8. Produit médical (100) selon l'une quelconque des revendications précédentes, **caractérisé en ce que** certains organes (110), en particulier les organes (110) au niveau des régions de bords, d'arêtes et/ou de coins du produit (100), présentent un plus grand diamètre intérieur que d'autres organes (110) du produit (100), en particulier les organes intérieurs (110) du produit.

9. Produit médical (100) selon l'une quelconque des revendications précédentes, **caractérisé en ce que** certains organes (110), en particulier les organes (110) au niveau des régions de bords, d'arêtes et/ou de coins du produit (100), présentent un marquage, en particulier un marquage de couleur, et/ou certains organes (110), en particulier les organes (110) au niveau des régions de bords, d'arêtes et/ou de coins du produit médical (100), présentent un élément de préhension et/ou certains organes (110), en particulier au niveau des régions de bords, d'arêtes et/ou de coins du produit (100) sont connectés les uns aux autres de telle sorte qu'ils ne puissent pas bouger les uns par rapport aux autres.

10. Produit médical (100) selon l'une quelconque des revendications précédentes, **caractérisé en ce que** les organes (110) présentent un matériau ou se composent d'un matériau qui est choisi parmi le groupe constitué de métaux, de polymères, de matériaux en céramique, de matériaux en ciment osseux, et de mélanges ou de combinaisons de deux ou de plus de deux des matériaux cités.

11. Produit médical (100) selon l'une quelconque des revendications précédentes, **caractérisé en ce que** le produit médical (100) présente en plus des organes (110), des éléments structurels supplémentaires (190).

12. Produit médical (100) selon la revendication 11, **caractérisé en ce que** les éléments structurels supplémentaires (190) se trouvent dans des espaces creux et/ou intermédiaires du produit médical (100).

13. Produit médical (100) selon l'une quelconque des revendications précédentes, **caractérisé en ce que** le produit (100) est un implant chirurgical, de préférence un substitut osseux.

14. Procédé de fabrication d'un produit médical (100) selon l'une quelconque des revendications précédentes, **caractérisé en ce qu'**une pluralité d'organes (110) avec une bordure périphérique (120) sont fabriqués et connectés les uns aux autres au moyen d'un procédé de fabrication additif ou génératif de telle sorte que les bordures (120) des organes connectés (110) viennent en prise les unes dans les autres.
